# FASCICULE DE BREVET EUROPEEN

(11) **EP 1 905 425 B1**
(45) Date de publication et mention de la délivrance du brevet: **24.07.2013**
(21) Numéro de dépôt: 07356128.4
(22) Date de dépôt: 26.09.2007
(51) Int. Cl.: A61K 8/14, A61K 8/55, A61K 8/92, A61Q 19/00, A23L 1/00, A61K 9/127, A23L 1/30, A61K 31/201

(54) **Vésicule inversée enfermant une phase hydrophobe**
Invertiertes Vesikel, das eine hydrophobe Phase einschließt
Reverse vesicle containing a hydrophobic phase

(30) Priorité: 26.09.2006 FR 0608458
(43) Date de publication de la demande: 02.04.2008
(73) Titulaire: St. Hubert, 54710 Ludres (FR)
(72) Inventeur: Dubois, Virginie, 54000 Nancy (FR); Marchal, Norbert, 54122 Flin (FR); Breton, Sylvie, 78000 Versailles (FR); Verdellet, Pierre, 75016 Paris (FR); Parmentier, Michel, 54740 Vaudeville (FR); Fanni, Jacques, 54600 Villiers lès Nancy (FR)
(74) Mandataire: Tripoz, Inès

(56) Documents cités:
- EP-A- 0 467 795
- EP-A2- 0 160 286
- WO-A-91/00084
- WO-A-95/22989
- FR-A- 2 771 635
- FR-A1- 2 735 658
- US-A- 4 804 539
- US-A- 5 015 483
- US-A- 5 139 803
- HIRONOBU KUNIEDA ET AL: "FORMATION OF REVERSED VESICLES" JOURNAL OF THE AMERICAN CHEMICAL SOCIETY, AMERICAN CHEMICAL SOCIETY, WASHINGTON, DC, US, vol. 113, no. 3, 30 janvier 1991 (1991-01-30), pages 1051-1052, XP000172752 ISSN: 0002-7863

## Description

La présente invention se rapporte à des vésicules tels que décrits dans la revendication 1 qui comprennent une cavité interne hydrophobe essentiellement dépourvue de solvant organique et au moins une bicouche constituée de deux couches de lipides amphiphiles déterminant entre elles un espace interstitiel hydrophile. L'invention décrit également leur procédé de préparation, ainsi que diverses utilisations.

Dans certaines industries comme l'industrie agro-alimentaire, cosmétique, pharmaceutique, l'introduction de composés d'intérêt actifs, hydrophobes ou hydrophiles, dans des phases grasses, peut se révéler difficile lorsque lesdits composés d'intérêt sont fragiles, instables, sensibles à l'oxydation. En effet, de nombreuses préparations agro-alimentaires, cosmétiques ou pharmaceutiques sont des produits constitués seulement d'une phase grasse comme les huiles ou des émulsions, eau dans huile ou huile dans eau, et il peut être nécessaire, en raison de l'instabilité de certains composés d'intérêt actifs, de les isoler de la phase aqueuse lorsqu'ils sont hydrophobes, et de les incorporer dans une phase grasse lorsqu'il s'agit de composés hydrophiles.
En effet, une solution, lorsque le composé d'intérêt est un composé hydrophobe, serait de l'introduire directement dans la phase grasse du produit, mais cette solution entraîne un contact possible avec la phase aqueuse lorsqu'il s'agit d'une émulsion.
Lorsque le composé d'intérêt est hydrophile, son incorporation est impossible si le produit est constitué seulement d'une phase grasse (cas des huiles...). De plus, lorsque le produit est une émulsion (eau dans huile, huile dans eau...), si ce composé est introduit dans la phase aqueuse du produit il pourrait être en contact avec la phase grasse du produit.
Aucune de ces solutions ne permet donc de résoudre le problème posé.

Il existe donc un besoin de moyens de protection et d'introduction de composés d'intérêt hydrophobes et/ou hydrophiles dans une phase grasse.

Dans l'art antérieur, des structures formées essentiellement de lipides ont donc été développées afin d'essayer d'isoler les composés d'intérêt. Les lipides sont de petites molécules hydrophobes principalement constituées de carbone, d'hydrogène et d'oxygène et ayant une densité inférieure à celle de l'eau. Ils peuvent être à l'état solide, comme dans les graisses, ou liquide, comme dans les huiles. Certains lipides comme les phospholipides sont en réalité des molécules amphiphiles qui présentent une « tête » polaire hydrophile et une « queue » apolaire hydrophobe. En solution, selon le type de solvant et le mode opératoire, les lipides amphiphiles s'orientent pour former divers types de structures : c'est ce qu'on appelle le polymorphisme lipidique.

Les lipides amphiphiles peuvent former par exemple des vésicules monocouches comme des micelles, qui sont des gouttelettes en suspension dans un milieu hydrophile comme l'eau, les « têtes » polaires des lipides étant orientées vers l'extérieur tandis que les « queues » apolaires sont orientées vers l'intérieur des vésicules qui renferme une phase hydrophobe.
Néanmoins, les micelles ne sont pas adaptées pour résoudre le problème technique qui se pose. En effet, même si l'incorporation d'un composé d'intérêt hydrophobe est possible dans la phase hydrophobe qu'elles renferment, les micelles doivent être introduites dans la phase aqueuse des produits du fait de leur caractère hydrophile. Leur introduction dans des produits constitués seulement d'une phase grasse (cas des huiles...) est donc impossible. Pour les produits qui sont des émulsions (eau dans huile, huile dans eau...), le composé d'intérêt de type hydrophobe (situé dans la phase hydrophobe) n'est pas suffisamment isolé de la phase aqueuse des produits, s'il est sensible à l'oxydation et/ou à l'hydrolyse. Concernant l'incorporation dans des micelles d'un composé d'intérêt hydrophile, cela est impossible.

D'autres structures ont été développées : les liposomes. En phase aqueuse, les lipides amphiphiles peuvent également former des feuillets stables dans lesquels les «têtes » polaires sont tournées vers la phase aqueuse et les « queues » apolaires dans le milieu des feuillets, lesquels sont aussi appelés bicouches ou films de Langmuir-Blodgett. Lorsqu'un tel feuillet se referme sur lui-même, on aboutit à la formation d'une vésicule dont la bicouche lipidique sépare la solution aqueuse externe (hydrophile) de la solution aqueuse interne (hydrophile) : c'est ce qu'on appelle un liposome. Selon leur nombre de bicouches lipidiques, les liposomes peuvent être uni- ou multilamellaires.

Des composés hydrophobes peuvent être incorporés dans les liposomes : ils sont alors non pas encapsulés dans la cavité interne hydrophile, mais à l'intérieur de la bicouche lipidique qui contient une phase hydrophobe. Par exemple, le brevet US 5,653,998 divulgue des formulations stables de liposomes renfermant des substances actives lipophiles et faiblement solubles appartenant notamment au groupe des dihydropyridines. Les liposomes permettent de protéger ces substances de l'hydrolyse et de l'oxydation, quand elles y sont sensibles.

Le brevet US 5,139,803 de Nabisco a pour objectif d'introduire des additifs alimentaires qui sont facilement oxydables et qui développent à court terme une mauvaise odeur et un mauvais goût, telles que les huiles végétales ou les huiles de poisson contenant notamment des oméga 3, dans des produits alimentaires, de telle sorte que ces additifs ne subissent pas l'oxydation. Ces substances oxydables sont alors introduites dans la bicouche lipidique des liposomes, ce qui empêche leur oxydation. On peut ensuite incorporer les liposomes dans des préparations alimentaires ayant un fort taux de matière grasse, mais à condition que ces préparations alimentaires soient constituées d'une phase aqueuse, continue ou dispersée (comme par exemple des margarines et autres émulsions eau dans huile).

De plus, si l'on souhaite également incorporer un composé d'intérêt hydrophile, cela est possible dans la cavité interne hydrophile des liposomes. C'est le cas, par exemple, dans US 4,883,665 divulguant un procédé de préparation de liposomes encapsulant des médicaments hydrophiles.

Néanmoins, les liposomes ne permettent pas non plus de résoudre le problème posé pour les mêmes raisons que les micelles. En effet, outre le fait que leur stabilité apparaît imparfaite puisque, avec le temps, on observe des phénomènes de coalescence ou de fuite, les liposomes doivent également être introduits dans la phase aqueuse des produits du fait de leur caractère hydrophile.
Leur introduction dans des produits constitués seulement d'une phase grasse (huile...) est donc impossible. Pour les produits qui sont des émulsions (eau dans huile, huile dans eau...), le composé d'intérêt de type hydrophobe (situé à l'intérieur de la bicouche lipidique) n'est pas suffisamment isolé de la phase aqueuse des produits, s'il est sensible à l'oxydation et/ou à l'hydrolyse.

D'autres structures monocouches comme les micelles inversées, gouttelettes en suspension dans un solvant non polaire hydrophobe, avec une orientation inversée des lipides amphiphiles, renfermant une phase interne hydrophile, ne conviennent pas non plus pour résoudre le problème technique posé. En effet, même si leur incorporation dans des produits constitués seulement d'une phase grasse (huile...) ou dans des produits qui sont des émulsions (eau dans huile, huile dans eau...) est possible du fait de leur caractère hydrophobe, ils ne peuvent pas incorporer des composés d'intérêts hydrophobe, car leur cavité interne est hydrophile.

Un article, du 30 janvier 1991, intitulé « Formation of reversed vesicles » de Hironobu Kunieda et al. , extrait de la revue Journal of the American Chemical Society (Vol. 113, n°3), fait état des recherches concernant la formation de vésicules lipidiques ayant une structure inversée par rapport à celle des liposomes, à savoir que les parties hydrophiles des lipides amphiphiles sont orientées tête-à-tête vers l'intérieur de la bicouche. Toutefois, la cavité interne de ces vésicules comprend un solvant organique : le dodécane ; ce qui empêche toute utilisation de ces vésicules lipidiques dans le domaine de l'alimentaire.

La présente invention permet de répondre au problème technique en proposant de nouvelles structures lipidiques, de type liposome à structure inversée, mais essentiellement dépourvues de solvant organique, et pouvant incorporer au moins un composé d'intérêt hydrophobe, et/ou au moine un composé d'intérêt hydrophile, et pouvant être introduites dans la phase grasse d'un produit constitué seulement d'une phase grasse (huile...) ou d'un produit qui est une émulsion (eau dans huile, huile dans eau...), et également leur procédé de préparation, ainsi que diverses utilisations.

Les structures de la présente invention sont des vésicules lipidiques à structure de liposome inversée selon la revendication 1, et caractérisées en ce que chaque vésicule comprend une cavité interne hydrophobe essentiellement dépourvue de solvant organique et au moins une bicouche constituée de deux couches de lipides amphiphiles déterminant entre elles un espace interstitiel hydrophile.

Par cavité interne hydrophobe essentiellement dépourvue de solvant organique, on comprend selon invention une quantité de solvant organique n'excédant pas celle compatible avec les utilisations indiquées, par exemple cosmétiques ou pharmaceutiques. Par contre, dans certaines applications, notamment alimentaires, la cavité interne hydrophobe sera totalement exemple de solvant organique.

Ainsi, il est décrit des structures de type vésicules lipidiques à structure de liposome inversée, et caractérisées en ce que chaque vésicule comprend une cavité interne hydrophobe dépourvue de solvant organique et au moins une bicouche constituée de deux couches de lipides amphiphiles déterminant entre elles un espace interstitiel hydrophile.

Du fait de leur caractère hydrophobe, les vésicules, objets de la présente invention, sont facilement incorporables dans des phases grasses. De plus, ces vésicules peuvent incorporer des composés d'intérêt hydrophobes dans la phase hydrophobe (et ainsi les isoler de la phase aqueuse lorsque les produits les incorporant sont des émulsions) et/ou des composés d'intérêt hydrophiles. Ces derniers sont alors situés dans l'espace interstitiel hydrophile déterminé par la au moins une bicouche de lipides amphiphiles.

Ci-après, dans la description et les revendications de la présente demande, lorsqu'il sera fait mention de la bicouche de lipides amphiphiles, cela se rapportera à toutes les vésicules objets de la présente invention : à savoir aussi bien celles comprenant une seule bicouche que celles comprenant plusieurs bicouches.

Dans la présente demande, par composés d'intérêt, on entend des composés hydrophobes ou hydrophiles qui sont « à protéger » (car ils sont sensibles à l'oxygène, à l'eau...), c'est-à-dire qu'il faut limiter leur contact avec soit la phase aqueuse pour les composés d'intérêt hydrophobes ou soit la phase grasse pour les composés d'intérêt hydrophiles, et/ou des composés hydrophobes ou hydrophiles qui sont « à délivrer », à savoir des substances actives tels que par exemples des principes actifs ou ingrédients pharmaceutiques et/ou cosmétiques, ou encore des principes actifs ou des ingrédients alimentaires.

Parmi les composés d'intérêt hydrophobes, on peut citer :
- les principes actifs ou ingrédients pharmaceutiques et/ou cosmétiques hydrophobes pouvant être choisis parmi le paracétamol, le pindolol, le probucol,
- les principes actifs ou ingrédients alimentaires hydrophobes pouvant être choisis parmi les antioxydants (extrait de romarin, de thé vert, mélange de tocophérols), les acides gras libres (pouvant être choisis parmi l'EPA, le DHA, l'acide arachidonique... ou des acides gras essentiels comme l'acide linoléique, l'acide alpha-linolénique), les stérols végétaux, les stanols végétaux, les polycosanols, les vitamines liposolubles (A, D, E, K), les colorants (caroténoïdes, flavonoïdes...), les composés aromatiques (diacétyle...), les huiles alimentaires aromatiques (huiles vierges de noix, d'olive, de noisette...) ou encore parmi d'autres huiles telles que :

- les huiles essentielles (de citron, de lavande, d'eucalyptus, de camphre, de sauge, de thym, de girofle...),
- les huiles polyinsaturées marines comme les huiles de poisson, de crustacé ou d'algues
- les huiles polyinsaturées végétales.
Celles-ci peuvent être choisies parmi les huiles polyinsaturées végétales riches en acides gras oméga 3 comme les huiles d'amarante (*Amaranthus viridis*), d'argousier (*Hippophae rhamnoides*), de bélize (*Ximenia americana*), de cameline (*Camelina sativa*), de chanvre (*Cannabis sativa*), de chia (*Salvia hispanica*)*,* de colza (*Brassica napus*), d'échium (*Echium sp.*)*,* de fenugrec (*Trigonella foenum-graecum*), de lin (*Linum usitatissimum*), de matthiole (*Matthiola tricuspidata*), de moutarde blanche (*Brassica alba*), de noix (*Juglans regia*), de pépin de canneberge (Vaccinium *oxycoccos*), de pépin de cassis (*Ribes nigrum*)*,* de pépin de framboise (*Rubus idaeus*), de perilla (*Perilla frutescens*)*,* de pourpier (*Portulaca oleracea*), de salicorne (*Salicornia europaea*).
Les huiles polyinsaturées végétales peuvent aussi être choisies parmi les huiles polyinsaturées végétales riches en acides gras oméga 6 comme les huiles d'amarante (*Amaranthus caudatus ; Amaranthus cruentus ou Amaranthus hypocondriacus*), d'argan (*Argania spinosa*), d'artichaut *(Cynara scolymus*), d'avoine (*Avena sativa*), de bourrache (*Borago officinalis*), de cactus (*Opuntia ficus indica*), de carthame (*Carthamus tintorius*), de cumin (*Cuminum cymimum),* de cumin noir (*Nigella sativa*), de germe de blé (*Triticum aestivum*), de graine de coton (*Gossypium hirsutum*)*,* de graine de paprika *(Capsicum annuum),* de graine de tabac (*Nicotiana tabacum*)*,* d'haricot africain (*Pentaclethra macrophylla*), de kénaf (*Hibiscus cannabinus*)*,* de lupin (*Lupinus mexicanus*), de maïs (*Zea mais*), de Niger (Guizotia abyssinica), de noyer cendré (Juglans cinerea), d'oignon (*Allium cepa*), d'onagre (*Oenothera biennis*), de pépin de citrouille (*Cucurbita pepo*)*,* de pépin de melon (*Cucumis melo*), de pépin de pastèque (*Citrullus sp.*), de pépin de raisin (*Vitis vinifera*), de quinoa (*Chenopodium quinua*), de ratanjyot (*Jatropha curcas*), de salicorne (*Salicornia bigelovii*), de sarrasin (*Polygonum fagopyrum*), de sésame (*Sesamum indicum*), de soja (*Glycine max*), de son de riz (*Oryza sativa*), de tournesol (*Helianthus annuus*).

Parmi les composés d'intérêt hydrophiles, on peut citer :
- les principes actifs ou ingrédients pharmaceutiques et/ou cosmétiques hydrophiles pouvant être choisis parmi les antitumoraux (adriamycine, actimycine, mitomycine, 1 β-arabinofuranosyl cytosine, cisplatine), les antiviraux (interféron), les aminoglucosides (gentamicine), les antibiotiques (β-lactam, sulbenicilline, céfotiame, cefmenoxime), les hormones peptidiques (TRH, leuprolide, insuline), les agents immunopotentialisateurs (muramyldipeptide, muramyltripeptide), les protéines (immunoglobulines, toxines), les agents anti-allergiques, antimycotiques, cytostatiques ou de diagnostic, les anxiolytiques, les contraceptifs, les sédatifs.
- les principes actifs ou ingrédients alimentaires hydrophiles pouvant être choisis parmi les sels minéraux (calcium, chlore, magnésium, phosphore, potassium, sodium, soufre), les oligo-éléments (aluminium, brome, cuivre, cobalt, fer, fluor, manganèse, molybdène, iode, sélénium, silicium, vanadium, zinc), les glucides (comme le glucose, le galactose, le mannose, le maltose, le maltotriose), les acides aminés (alanine, arginine, asparagine, acide aspartique, cystéine, acide glutamique, glutamine, glycine, histidine, isoleucine, leucine, lysine, méthionine, phénylalanine, proline, sérine, thréonine, tryptophane, tyrosine, valine), les peptides, les protéines, les vitamines hydrosolubles, les arômes (de citron, de vanille, de fraise, de café, de noisette, de framboise...), les polyols.

Lorsque les composés d'intérêt sont hydrophobes, ils vont être incorporés (seuls ou en mélange) dans la cavité interne hydrophobe des vésicules. Lorsque les composés d'intérêt sont hydrophiles, ils vont être incorporés (seuls ou en mélange) dans l'espace interstitiel hydrophile déterminé par la bicouche de lipides amphiphiles.

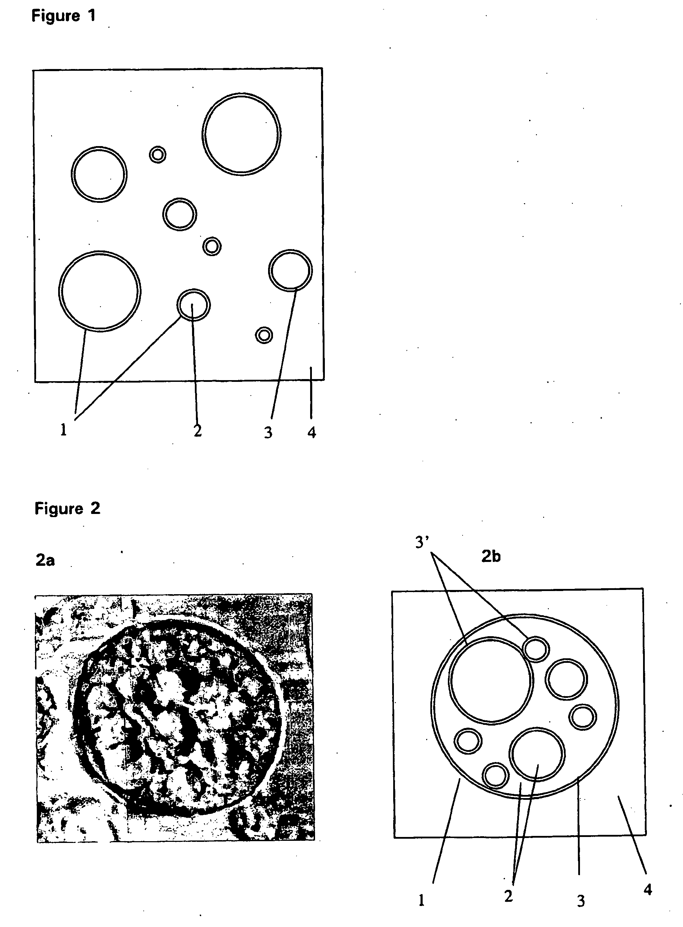

La figure 1 est une représentation schématique des vésicules selon la présente invention qui ne comportent qu'une seule bicouche de lipides amphiphiles, séparant la cavité interne hydrophobe de la phase grasse du produit dans lequel sont incorporées lesdites vésicules. Cette bicouche représente ainsi la membrane desdites vésicules.

La figure 2 représente une vésicule selon la présente invention qui comporte plusieurs bicouches de lipides amphiphiles qui sont juxtaposées à l'intérieur de la bicouche séparant la cavité interne hydrophobe de la phase grasse du produit dans lequel est incorporée ladite vésicule :
2a est une observation microscopique
2b est une représentation schématique

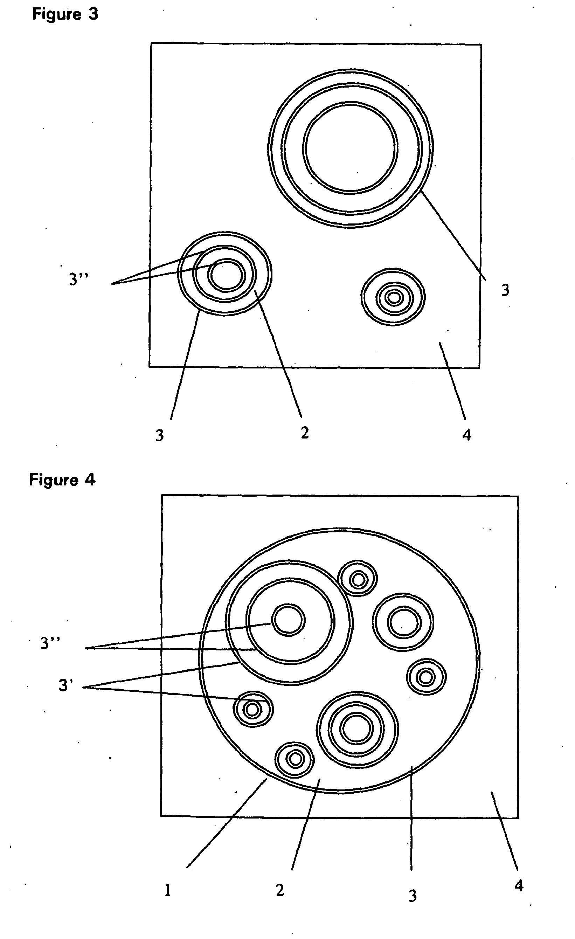

La figure 3 est une représentation schématique de vésicules selon la présente invention, qui comportent plusieurs bicouches de lipides amphiphiles qui sont concentriques à la bicouche séparant la cavité interne hydrophobe de la phase grasse du produit dans lequel sont incorporées lesdites vésicules. Ce type de structure est également qualifié de « multilamellaire ».

La figure 4 est une représentation schématique d'une vésicule selon la présente invention, dont les bicouches de lipides amphiphiles sont à la fois juxtaposées et concentriques à l'intérieur de la bicouche séparant la cavité interne hydrophobe de la phase grasse du produit dans lequel est incorporée ladite vésicule.

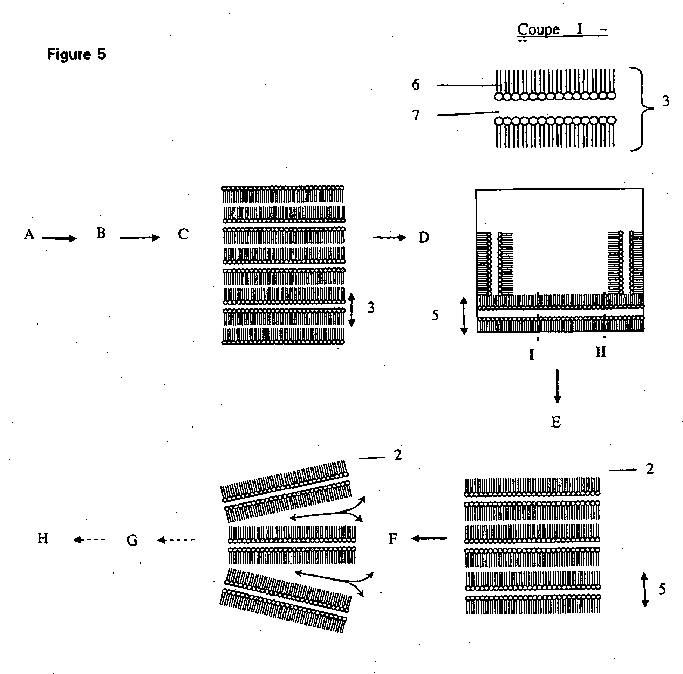

La figure 5 est une représentation schématique d'un exemple de procédé de préparation de vésicules selon la présente invention.

La présente invention concerne des vésicules comportant une seule bicouche de lipides amphiphiles comme illustrées à la figure 1 de la description, ainsi que des vésicules comportant plusieurs bicouches de lipides amphiphiles comme illustrées aux figures 2, 3 et 4.

D'après la figure 1, la vésicule (1) enferme une cavité interne hydrophobe (2) qui est séparée de la phase grasse (4) du produit dans lequel est incorporée ladite vésicule (1) par une bicouche (3) formée de lipides amphiphiles déterminant un espace interstitiel hydrophile.

Les vésicules selon la présente invention peuvent également comporter plusieurs bicouches (3, 3', 3") comme l'illustrent les figures 2, 3 et 4.
Une vésicule (1) peut comprendre plusieurs bicouches (3, 3'), dont l'une (3) sépare la cavité interne hydrophobe (2) de la phase grasse (4), et les autres bicouches (3') sont juxtaposées à l'intérieur de ladite vésicule (1) (figure 2). On peut ainsi avoir des bicouches (3') juxtaposées à partir d'un nombre total de trois bicouches (3, 3') de la vésicule (1).

Dans un autre mode de réalisation de l'invention, les autres bicouches (3") sont concentriques entre elles et à la bicouche (3) qui sépare la cavité interne hydrophobe (2) de la phase grasse (4) (figure 3). On peut ainsi avoir des bicouches (3") concentriques à partir d'un nombre total de deux bicouches (3, 3") de la vésicule (1).

Dans un autre mode de réalisation de l'invention, la vésicule (1) peut comprendre plusieurs bicouches (3, 3', 3"), dont l'une (3) sépare la cavité interne hydrophobe (2) de la phase grasse (4), et les autres bicouches (3', 3") sont juxtaposées et concentriques à l'intérieur de ladite vésicule (1) (figure 4). On peut ainsi avoir des bicouches (3', 3") juxtaposées et concentriques à partir d'un nombre total de 4 bicouches (3, 3', 3") de la vésicule (1).

Les vésicules (1) peuvent être sphériques ou fusiformes selon leur formulation. Lorsqu'elles sont sphériques, leur taille est comprise entre 25 et 100 µm, et généralement inférieure à 50 µm. Lorsqu'elles sont fusiformes, leur largeur est comprise entre 25 et 50 µm, et leur longueur entre 75 et 100 µm.

Chaque vésicule (1) comprend une cavité interne hydrophobe (2) et au moins une bicouche (3) constituée de deux couches de lipides amphiphiles (6) déterminant entre elles un espace interstitiel hydrophile (7), illustré à la figure 5.

Dans un mode de réalisation, les vésicules de la présente invention se trouvent dans la phase grasse (4) d'un produit. Dans le cas d'une émulsion, cette phase grasse (4) peut être continue ou discontinue.

Contrairement aux structures des liposomes, dans la présente invention, les lipides amphiphiles de la bicouche (3, 3', 3") ont leur partie hydrophile (« tête » polaire) orientée tête-à-tête vers l'intérieur de cette bicouche (3, 3', 3") et déterminent ainsi un espace interstitiel hydrophile (7). Par voie de conséquence, leur partie hydrophobe (« queue » apolaire) est orientée vers l'extérieur de cette bicouche (3,3', 3"). Ces vésicules sont donc facilement intégrables dans la phase grasse (4) d'un produit constitué seulement d'une phase grasse (huile...) ou dans la phase grasse (4) continue ou discontinue d'un produit qui est une émulsion (eau dans huile, huile dans eau, émulsions triples...).

Dans un mode de réalisation particulier, les lipides amphiphiles (6) permettant de former la bicouche (3) sont des phospholipides. Les phosphatidylcholines sont en particulier choisis, et notamment les lécithines de soja.

Ces lipides amphiphiles (6) permettant de former la bicouche (3) peuvent être utilisés seuls.

Dans un mode de réalisation particulier, au moins un émulsifiant peut être additionné à ces lipides amphiphiles. Cet émulsifiant peut par exemple être choisi parmi le groupe des monoglycérides ou des diglycérides d'acides gras. Les émulsifiants peuvent être des esters citriques d'acides gras.

Dans un mode de réalisation particulier, les lipides amphiphiles permettant de former la bicouche (3) peuvent être stabilisés par au moins un composé stabilisateur. Il peut s'agir par exemple d'un composé de type tocophérol comme l'alpha-tocophérol, ou de type stérol comme le cholestérol.

Dans un autre mode de réalisation, à la fois au moins un émulsifiant et au moins un composé stabilisateur sont ajoutés aux lipides amphiphiles formant la bicouche (3).

Les deux couches de lipides amphiphiles (6) formant la bicouche (3) déterminent un espace interstitiel hydrophile (7), ledit espace représentant la phase hydrophile des vésicules (1) de la présente invention.

Dans un mode particulier de réalisation, cet espace interstitiel hydrophile (7) est constitué d'eau.

Dans un mode de réalisation particulier, au moins un agent faisant barrière à l'oxygène est en outre présent dans cet espace interstitiel hydrophile (7). Parmi les agents faisant barrière à l'oxygène, on peut citer les gels d'hydrocolloïdes peu hydratés, la cire d'abeille, de l'isolat de protéines de lactosérum, de l'isolat de protéines de soja, de la β -lactoglobuline, de la caséine, des sels de caséine comme le caséinate de sodium, du sorbitol, du glycérol. Ceux-ci peuvent être utilisés seuls ou en mélange.

Dans un autre mode de réalisation, au moins un agent capable d'augmenter la rigidité de la bicouche (3) et/ou de diminuer la disponibilité de la phase hydrophile (qui peut être constituée d'eau) est en outre présent dans l'espace interstitiel hydrophile (7). Cet agent peut être choisi parmi les hydrocolloïdes. On peut citer, comme exemple d'hydrocolloïdes, l'amidon natif (de blé, de riz, de maïs, de paprika, de pomme de terre...), l'amidon réticulé, l'amidon prégélatinisé, l'amylopectine ou alors les produits de dégradation de l'amidon comme les maltodextrines de différents degrés d'hydrolyse, ou encore des gommes. Des exemples de gommes sont les gommes d'acacia, les gommes guar, les gommes xanthane, les gommes adragante, les gommes karaya, les gommes tara, les gommes gellane.
Les agents capables d'augmenter la rigidité de la bicouche (3) de lipides amphiphiles (6) et/ou de diminuer la disponibilité de la phase hydrophile peuvent également être choisis parmi le sorbitol, le sirop de sorbitol, le mannitol, le glycérol, le glucomannane, le stéarate de polyoxyéthylène (8), le stéarate de polyoxyéthylène (40), le monolaurate de polyoxyéthylène sorbitane (polysorbate 20), le monooléate de polyoxyéthylène de sorbitane (polysorbate 80), le monopalmitate de polyoxyéthylène sorbitane (polysorbate 40), le monostéarate de polyoxyéthylène sorbitane (polysorbate 60), le tristéarate de polyoxyéthylène sorbitane (polysorbate 65), la pectine, la pectine amidée, les phosphatides d'ammonium, l'acétate isobutyrate de saccharose, les esters glycériques de résine de bois, l'acide alginique, les alginates de sodium, de potassium, d'ammonium, de calcium ou de propane-1,2-diol, l'agar-agar, les carraghénanes, les algues euchema transformées ou la farine de graines de caroube.
Tous ces exemples d'agents capables d'augmenter la rigidité de la bicouche (3) et/ou de diminuer la disponibilité de la phase hydrophile peuvent être utilisés seuls ou en mélange.

Dans un mode particulier de réalisation, à la fois au moins un agent faisant barrière à l'oxygène et au moins un agent capable d'augmenter la rigidité de la bicouche (3) et/ou de diminuer la disponibilité de la phase hydrophile sont présents dans l'espace interstitiel hydrophile (7).

Par exemple, la phase hydrophile est constituée d'eau à laquelle du caséinate de sodium et des gommes xanthane sont ajoutés.

Dans un mode de réalisation particulier, l'espace interstitiel hydrophile peut en outre comprendre au moins un composé d'intérêt hydrophile. Il s'agit des composés précédemment cités.

Chaque vésicule (1) de la présente invention enferme une cavité interne hydrophobe (2).

Dans un mode de réalisation, ladite cavité interne hydrophobe (2) est constituée d'au moins un composé neutre hydrophobe, c'est-à-dire qu'on ne cherche ni à le protéger, ni à le délivrer.

Dans un autre mode de réalisation, la cavité interne hydrophobe (2) est constituée d'au moins un composé d'intérêt hydrophobe (« à protéger » et/ou « à délivrer »).

Dans un autre mode de réalisation, la cavité interne hydrophobe (2) est constituée à la fois d'au moins un composé neutre hydrophobe et d'au moins un composé d'intérêt hydrophobe.

Dans un mode de réalisation particulier, la cavité interne hydrophobe (2) est essentiellement dépourvue de solvant organique.

Dans un mode de réalisation particulier, la cavité interne hydrophobe (2) est dépourvue de solvant organique.

La cavité interne hydrophobe (2) est huileuse et est constituée d'au moins un composé neutre hydrophobe huileux, c'est-à-dire qu'on ne cherche ni à le protéger, ni à le délivrer.
Le composé neutre hydrophobe est une huile neutre choisie parmi les huiles d'arachide *(Arachis hypogea),* d'adjouaba *(Haematostophis barten),* d'avocat *(Persea americana),* de babassu *(Orbignya oleifera),* de beurre de karité *(Vitellaria paradoxe),* de beurre de cacao *(Theobroma cacao),* de chufa *(Cyperus esculentus),* de crambe *(Crambe abyssinica),* de cuphea *(Cuphea tolucana, Cuphea wrightii, Cuphea laminuligena, Cuphea ilavea ou Cuphea leptopoda),* d'érable (*Acer saccharum* ou Acer saccharinum), de Gokhru (*Tribulus terrestris*), de noisette (*Corylus avellana*), de noix de coco (*Cocus nucifera*), de noix de Macadamia (*Macadamia tecraphylla),* de noix du Cambodge (*irvingia malayana*), de noix pili (*Canarium ovatum*), de noyau d'abricot *(Prunus armeniaca*), d'olive (*Olea europaea*), de palme (*Elaeis guineensis*), de palmiste (*Elasis guineensis*), de pépine de mangue (*Mangifer indica*), de pistache (*Pistacis atlantica*), ou des fractions de ces huiles (comme par exemple les oléines et stéarines de palme ou de palmiste). Ces huiles neutres peuvent être utilisées seules ou en mélange.

Dans un autre mode de réalisation, la cavité interne hydrophobe (2) comprend en outre au moins un antioxydant qui protège les composés d'intérêt hydrophobes qui sont sensibles à l'oxydation. Cet antioxydant peut être différent des antioxydants cités dans les exemples de composés d'intérêt hydrophobes.

Cet antioxydant peut être choisi parmi tous les composés ayant des propriétés antioxydantes connues. Il peut s'agir par exemple de tocophérols (mélange naturel, alpha-tocophérol de synthèse, gamma-tocophérol de synthèse, delta-tocophérol de synthèse), du palmitate d'ascorbyle, du stéarate d'ascorbyle, du gallate de propyle, du gallate d'octyle, du gallate dodécyle, du BHA (buthylhydroxyanisol), du BHT (buthylhydroxytoluène), de l'EDTA (éthylène diamine tétra acétate), de l'extrait naturel de romarin, de l'extrait de thé vert, des polyphénols. Ces exemples d'antioxydants peuvent être utilisés seuls ou en mélange.

Ci-après, dans la description et les revendications de la présente demande, les pourcentages massiques mentionnés seront exprimés par rapport à la masse totale de la vésicule (1).

Dans une formulation de vésicule (1) selon la présente invention, les pourcentages massiques peuvent être les suivants :
- le pourcentage massique de la cavité interne hydrophobe (2) est compris entre 70 et 89%,
- le pourcentage massique de la bicouche (3) de lipides amphiphiles (6) est compris entre 10 et 22,5%,
- le pourcentage massique de l'espace interstitiel hydrophile (7) contenu dans la bicouche (3) est compris entre 3,8 et 8,5%.

Dans une formulation, la cavité interne hydrophobe (2) est constituée d'au moins un composé neutre hydrophobe et/ou d'au moins un composé d'intérêt hydrophobe représentant de 70 à 89% en pourcentage massique par rapport à la masse totale de la vésicule (1), et comprend en outre au moins un antioxydant représentant de 0 à 4% en pourcentage massique.

Dans une formulation, la bicouche (3) est constituée de lipides amphiphiles (6) représentant de 10 à 22,5% en pourcentage massique par rapport à la masse totale de la vésicule (1), et comprend en outre au moins un émulsifiant représentant de 0 à 4,5% en pourcentage massique, et comprend en outre au moins un stabilisateur représentant de 0 à 1 % en pourcentage massique.

Dans une formulation, l'espace interstitiel hydrophile (7) est constitué d'eau représentant de 3,8 à 8,5% en pourcentage massique. Dans une formulation, il est constitué de 4 % d'eau.

Dans une formulation, l'espace interstitiel hydrophile (7) est constitué d'eau représentant de 3,8 à 8,5% en pourcentage massique, et il comprend en outre au moins un agent faisant barrière à l'oxygène et/ou un agent capable d'augmenter la rigidité de la bicouche (3) et/ou de diminuer la disponibilité de la phase hydrophile, représentant de 0 à 2,335% en pourcentage massique.

Dans une formulation, l'espace interstitiel hydrophile (7) est constitué d'eau représentant de 3,8 à 8,5% en pourcentage massique, et il comprend en outre au moins un agent faisant barrière à l'oxygène représentant de 0 à 0,2% en pourcentage massique, et/ou au moins un agent capable d'augmenter la rigidité de la bicouche lipidique et/ou de diminuer la disponibilité de la phase hydrophile représentant de 0 à 2,135% en pourcentage massique.

Dans une formulation répondant à celle ci-dessus, l'espace interstitiel hydrophile (7) comprend :
- de l'amidon natif ou réticulé représentant de 0 à 1,5% en pourcentage massique,
- et/ou des maltodextrines représentant de 0 à 0,3% en pourcentage massique,
- et/ou de la gomme acacia représentant de 0 à 0,3% en pourcentage massique,
- et/ou de la gomme guar représentant de 0 à 0,02% en pourcentage massique,
- et/ou de la gomme xanthane représentant de 0 à 0,015% en pourcentage massique.
Dans une autre formulation :
- la cavité interne hydrophobe (2) est constituée d'au moins un composé neutre hydrophobe et/ou d'au moins un composé d'intérêt hydrophobe représentant 75% en pourcentage massique par rapport à la masse totale de la vésicule (1) et d'au moins un antioxydant dont le pourcentage massique est de 3,5%,
- la bicouche (3) est constituée de lipides amphiphiles (6) représentant de 13 à 16% en pourcentage massique, et comprend au moins un émulsifiant représentant 3,5% en pourcentage massique, et au moins un stabilisateur représentant 0,8% en pourcentage massique,
- l'espace interstitiel hydrophile (7) est constitué d'eau représentant 4% en pourcentage massique, il comprend au moins un agent faisant barrière à l'oxygène représentant 0,11% en pourcentage massique, et il comprend au moins un agent capable d'augmenter la rigidité de la bicouche lipidique et/ou de diminuer la disponibilité de la phase hydrophile, représentant 0,705% en pourcentage massique.

Dans une formulation répondant à celle ci-dessus, l'espace interstitiel hydrophile (7) comprend:
- de l'amidon natif ou réticulé représentant 0,08% en pourcentage massique,
- des maltodextrines représentant 0,3% en pourcentage massique,
- de la gomme acacia représentant 0,3% en pourcentage massique,
- de la gomme guar représentant 0,015% en pourcentage massique,
- de la gomme xanthane représentant 0,01% en pourcentage massique.

La présente invention porte aussi sur toute phase grasse (4) d'un produit comprenant au moins une des vésicules (1) de la présente invention. Cette phase grasse (4) peut être la phase grasse continue ou discontinue d'une émulsion.

Les vésicules (1) de la présente invention peuvent être introduites dans des produits alimentaires, cosmétiques ou pharmaceutiques. Elles sont introduites dans la phase grasse (4) de ces produits.
Lorsque les vésicules (1) sont introduites dans des produits alimentaires, la cavité interne hydrophobe (2) desdites vésicules (1) est dépourvue de solvant organique.
Ces produits peuvent être constitués seulement d'une phase grasse (c'est le cas de l'huile...) ou il peut s'agir d'une émulsion (eau dans huile, huile dans eau...), laquelle peut être multiple, comme par exemple une émulsion triple (E/H/E ou H/E/H). Il peut aussi s'agir d'une dispersion du type lotion pouvant être biphasée.

Ces produits alimentaires, cosmétiques ou pharmaceutiques peuvent se présenter sous la forme solide, liquide ou semi liquide.

Dans un mode de réalisation particulier, les vésicules de la présente invention peuvent être introduites dans des huiles, du beurre ou des margarines. Pour ces produits, la cavité interne hydrophobe de ces vésicules est dépourvue de solvant organique.

Dans un autre mode de réalisation, ces vésicules peuvent être introduites dans des crèmes comme des crèmes de soins, des crèmes solaires, dans des laits, des gels ou des lotions.

Dans un autre mode de réalisation, ces vésicules peuvent être introduites dans des produits pharmaceutiques, quelle que soit leur forme gallénique (comprimés, solutions injectables, solutions buvables, gels ou crèmes).

Une variante de l'invention pourrait consister à introduire les vésicules de la présente invention dans la phase aqueuse des produits alimentaires, cosmétiques ou pharmaceutiques en comprenant une (à savoir les produits constitués seulement d'une phase aqueuse ou les produits comme les émulsions ayant une phase aqueuse continue ou discontinue), à condition de les recouvrir d'une couche hydrophile en les émulsionnant par exemple dans une solution contenant un polymère, puis de leur faire subir une technique classique de microencapsulation pour qu'elles se retrouvent dans une molécule encapsulante hydrophile.

La présente invention concerne également un procédé de préparation des vésicules (1) objets de l'invention. Un mode particulier de procédé est illustré à la figure 5.

Le procédé comprend les étapes suivantes :
- étape A : étalement des lipides amphiphiles (6),
- étape B : addition d'une phase hydrophile dont sera constitué l'espace interstitiel hydrophile (7),
- étape C : pose pour obtenir des bicouches lipidiques (3)
   Dans un mode de réalisation, le temps de pose est inférieur à 15 minutes. Dans un autre mode de réalisation, le temps de pose est compris entre 8 et 15 minutes.
- étape D : élimination de l'excès de phase hydrophile pour obtenir des « films phospholipidiques hydratés » (5),
   Comme le démontre la coupe I-II de l'un de ces « films phospholipidiques hydratés » (5), ceux-ci comprennent de part et d'autre une couche de lipides amphiphiles (6) dont les « têtes » polaires sont orientées tête-à-tête vers l'intérieur de la bicouche (3) et déterminent ainsi un espace interstitiel hydrophile (7), représentant la phase hydrophile des vésicules (1).
- étape E : addition d'une phase hydrophobe dont sera constitué la cavité interne hydrophobe huileuse (2) des vésicules (1),
   Dans un mode de réalisation, la phase hydrophobe est essentiellement dépourvue de solvant organique.
   Dans un autre mode de réalisation, la phase hydrophobe est dépourvue de solvant organique.
   Dans un autre mode de réalisation, la phase hydrophobe est huileuse.
- étape F : agitation forte
   Dans un mode de réalisation, le temps d'agitation est inférieur à 15 minutes. Dans un autre mode de réalisation, il est compris entre 5 et 10 minutes. L'agitation permet l'infiltration de la phase hydrophobe entre les « films phospholipidiques hydratés » (5) et le décollement de ces bicouches (3) contenant une phase hydrophile, pour aboutir au final à la formation de vésicules (1) séparées de la phase grasse (4) par une bicouche (3) et comportant dans la cavité interne hydrophobe huileuse d'autres bicouches (3', 3").

Dans un mode de réalisation, l'étalement des lipides amphiphiles (6) dans l'étape A est effectué sur la paroi d'un réacteur en rotation ou sur une surface plane. Par réacteur on comprend tout récipient approprié, tels que entre autres une cuve, un ballon...

Dans un mode de réalisation, au niveau de l'étape B, la phase hydrophile est ajoutée dans le réacteur, ou elle atomisée sur la surface plane ou alors la surface plane est immergée dans un bain de phase hydrophile.

Dans un mode de réalisation, la phase hydrophobe, au niveau de l'étape E, est ajoutée dans le réacteur ou placée dans un récipient (bécher, cuve...) puis ajoutée par-dessus les « films phospholipidiques hydratés » (5).

Dans un mode particulier de réalisation, les lipides amphiphiles étalés dans l'étape A sont des phospholipides. Les phosphatidylcholines sont en particulier choisis, et notamment les lécithines de soja.

Les lipides amphiphiles permettant de former la bicouche (3) peuvent être utilisés seuls.

Dans un mode de réalisation particulier, au moins un émulsifiant peut être additionné à ces lipides amphiphiles (6). Cet émulsifiant peut être choisi parmi les exemples d'émulsifiant cités précédemment.

Dans un mode de réalisation particulier, les lipides amphiphiles (6) permettant de former la bicouche (3) peuvent être stabilisés par au moins un composé stabilisateur. Il peut s'agir des stabilisateurs cités précédemment.

Dans un autre mode de réalisation, à la fois au moins un émulsifiant et au moins un composé stabilisateur sont ajoutés aux lipides amphiphiles formant la bicouche (3).

Dans un mode de réalisation, la phase hydrophile ajoutée à l'étape B est constituée d'eau.

Dans un autre mode de réalisation particulier, au moins un agent faisant barrière à l'oxygène est ajouté à la phase hydrophile. Celui-ci peut être choisi parmi les exemples d'agents faisant barrière à l'oxygène cités précédemment.

Dans un autre mode de réalisation, au moins un agent capable d'augmenter la rigidité de la bicouche (3) et/ou de diminuer la disponibilité de la phase hydrophile est ajouté à la phase hydrophile. Celui-ci peut être choisi parmi les exemples d'agents capables d'augmenter la rigidité de la bicouche lipidique et/ou de diminuer la disponibilité de la phase hydrophile cités précédemment.

Dans un mode particulier de réalisation, à la fois au moins un agent faisant barrière à l'oxygène et au moins un agent capable d'augmenter la rigidité de la bicouche lipidique et/ou de diminuer la disponibilité de la phase hydrophile sont ajoutés à la phase hydrophile.

Par exemple, la phase hydrophile peut être constituée d'eau à laquelle du caséinate de sodium et des gommes xanthane sont ajoutés.

Dans un mode de réalisation, la phase hydrophile contenue dans la bicouche (3) peut en outre comprendre au moins un composé d'intérêt hydrophile.

Dans un mode de réalisation particulier, la phase hydrophobe qui est ajoutée à l'étape E est constituée d'au moins un composé neutre hydrophobe. Il peut s'agir d'une huile ou d'un mélange d'huiles neutres dont des exemples sont cités précédemment.

Dans un autre mode de réalisation particulier, la phase hydrophobe est constituée d'au moins un composé d'intérêt hydrophobe (« à protéger » et/ou « à délivrer »).
Dans un mode de réalisation, le composé d'intérêt hydrophobe est une huile de poisson.

Dans un mode de réalisation, la phase hydrophobe est constituée d'au moins un composé neutre hydrophobe et/ou d'au moins un composé d'intérêt hydrophobe.

Dans un mode de réalisation, au moins un antioxydant peut être additionné à la phase hydrophobe. Il peut s'agir des exemples cités précédemment, et peuvent être utilisés seuls ou en mélange.

Le procédé décrit ci-dessus peut comprendre une étape supplémentaire :
- étape G : décantation et récupération des vésicules.

Le procédé peut aussi comprendre une autre étape supplémentaire:
- étape H : introduction des vésicules dans la phase grasse (4) d'un produit.
   Lorsque le produit est une émulsion, cette phase grasse (4) est continue ou discontinue.

Le procédé ainsi décrit permet d'obtenir des vésicules (1) comme illustrées à la figure 1 et/ou des vésicules telles qu'illustrées aux figures 2,3, et 4. Le fait que les vésicules comprennent une ou plusieurs bicouches (3) dépend essentiellement de deux facteurs :
- de l'épaisseur des « films phospholipidiques hydratés » (5) obtenus à l'étape D (un film très fin permettra plutôt d'obtenir des vésicules (1) comportant une seule bicouche (3) délimitant lesdites vésicules (1) de la phase grasse (4)),
- et surtout de la vitesse d'agitation au niveau de l'étape F (une grande vitesse d'agitation permettra plutôt d'obtenir des vésicules comprenant plusieurs bicouches (3, 3', 3")).

En ce qui concerne la variante de l'invention consistant à introduire les vésicules (1) de la présente invention dans la phase aqueuse des produits alimentaires, cosmétiques ou pharmaceutiques en présentant une (à savoir les produits constitués seulement d'une phase aqueuse ou les produits comme les émulsions ayant une phase aqueuse continue ou discontinue), le procédé doit inclure des étapes supplémentaires :
- préparation d'une émulsion (par exemple dans une solution contenant un polymère) pour recouvrir les structures d'une couche hydrophile, et
- traitement par une technique classique de microencapsulation pour qu'elles se retrouvent dans une molécule encapsulante hydrophile.

Les vésicules (1) de la présente invention peuvent faire l'objet de multiples utilisations.

Dans un mode d'utilisation, ces vésicules peuvent être utilisées comme système transporteur-délivreur d'au moins un composé d'intérêt hydrophobe et/ou hydrophile.

Par système transporteur-délivreur, on entend la capacité qu'ont ces vésicules à transporter au moins un composé spécifique qui pourra éventuellement être ensuite libéré (« délivré ») dans des produits alimentaires, cosmétiques, pharmaceutiques incorporant ces vésicules (1), ou directement au niveau d'une cible comme par exemple un organe (organe digestif par exemple) ou un tissu du corps humain (comme la peau), au sein de laquelle le ou les composés spécifiques pourront éventuellement être absorbés.

Lorsque le composé d'intérêt est hydrophile, il se trouve dans l'espace interstitiel hydrophile (7) de la bicouche (3). Ledit composé d'intérêt hydrophile est choisi parmi les principes actifs ou ingrédients pharmaceutiques et/ou cosmétiques hydrophiles ou les principes actifs ou ingrédients alimentaires hydrophiles.

Lorsque le composé d'intérêt est hydrophobe, il peut être choisi parmi les principes actifs ou ingrédients pharmaceutiques et/ou cosmétiques hydrophobes ou les principes actifs ou ingrédients alimentaires hydrophobes, et il se trouve dans la cavité interne hydrophobe (2). Le composé d'intérêt hydrophobe peut être une huile choisie parmi les huiles essentielles, les huiles polyinsaturées marines comme les huiles de poisson ou les huiles polyinsaturées végétales),

Un autre mode d'utilisation des vésicules (1) de la présente invention concerne la protection contre l'hydrolyse et/ou l'oxydation d'au moins un composé d'intérêt, notamment quand il est hydrophobe.
Ce composé peut être un composé d'intérêt hydrophobe (comme une huile choisie parmi les huiles essentielles, les huiles polyinsaturées marines comme les huiles de poisson, de crustacé, d'algues ou les huiles polyinsaturées végétales) présent dans la cavité interne hydrophobe (2) des vésicules (1).

Un autre mode d'utilisation des vésicules (1) de la présente invention concerne l'enrichissement de produits ayant une phase grasse (4) en au moins un composé d'intérêt hydrophobe présentant un fort taux d'acides gras insaturés et/ou d'oméga 3/6 (comme une huile de poisson par exemple).

Ces produits enrichis peuvent être constitués seulement d'une phase grasse (c'est le cas des huiles...) ou il peut s'agir d'une émulsion (eau dans huile, huile dans eau, émulsion multiple comme une émulsion triple (E/H/E ou H/E/H)...) pour lesquelles la phase grasse (4) peut être continue ou discontinue. Les produits peuvent être des produits alimentaires comme du beurre ou des margarines, des produits cosmétiques ou pharmaceutiques.

Une autre application particulière consiste en l'utilisation de vésicules de la présente invention comprenant dans la cavité interne hydrophobe (2) un composé d'intérêt hydrophobe qui est une huile (comme une huile choisie parmi les huiles essentielles, les huiles polyinsaturées marines comme les huiles de poisson, de crustacé, d'algues ou les huiles polyinsaturées végétales), dans des produits alimentaires, cosmétiques ou pharmaceutiques, pour empêcher le développement d'odeurs et/ou de goûts désagréables dégagés par ladite huile.

Dans les exemples suivants, la cavité interne hydrophobe est huileuse. Toutefois, ces exemples sont donnés à titre d'illustration et n'ont en aucun cas un caractère limitatif.

### Exemple 1 : exemples de formulations

Des vésicules répondant aux dix formulations détaillées dans le tableau I ont été préparées en utilisant le procédé suivant :
- étalement de lipides amphiphiles formant la ou les bicouches lipidiques (3) sur la paroi d'un ballon en rotation. Les lipides amphiphiles choisis sont des phospholipides, et plus particulièrement des lécithines de soja auxquelles un émulsifiant et/ou un stabilisateur ont été ou non ajoutés selon les formulations.
- addition dans le ballon d'une phase hydrophile. La phase hydrophile utilisée ici est constituée d'eau pour les formulations 1 à 3 ou d'eau additionnée d'au moins un agent faisant barrière à l'oxygène et/ou un agent capable d'augmenter la rigidité de la bicouche lipidique et/ou de diminuer la disponibilité de l'eau, conformément à la présente invention, pour les formulations 4 à 10.
- pose.
   Les divers temps de pose sont les suivants :
   90 secondes pour la formulation 3 ;
   120 secondes pour les formulations 2, 4 et 5 ;
   150 secondes pour les formulations 6, 7, 9 et 10 ;
   210 secondes pour la formulation 8 ;
   300 secondes pour la formulation 1.
- élimination de l'excès de phase hydrophile et obtention de « films phospholipidiques hydratés » (5).
- addition dans le ballon d'une phase hydrophobe constituée d'un composé d'intérêt hydrophobe comme par exemple un AGPI (acide gras polyinsaturé) choisi parmi ceux mentionnés précédemment auquel est ajouté un antioxydant dans les formulations 2 à 10.
- agitation forte.
   Les divers temps d'agitation sont les suivants :
   60 secondes pour les formulations 2, 3 et 4 ;
   90 secondes pour la formulation 5 ;
   120 secondes pour les formulations 6 à 10 ;
   210 secondes pour la formulation 1.
- décantation et récupération des structures uni- ou multivésiculaires.

Dans le tableau, les valeurs des divers constituants sont exprimées en pourcentage massique (% m/m) par rapport à la masse totale de la structure.

**Tableau I :**

| **Ex** | **Lipides amphiphiles** | **Emulsifiant** | **Stabilisateur** | **Phase hydrophile** | **Autre composé de la phase hydrophile** | **Phase hydrophobe** | **Antioxydant** |
|---|---|---|---|---|---|---|---|
| **1** | Lécithine de soja 4,3 | - | - | Eau 4,1 | - | AGPI 81,6 | - |
| **2** | Lécithine de soja 16,9 | - | Alpha-tocophérol 0,8 | Eau 3,8 | - | AGPI 75,1 | Vit. E 3,4 |
| **3** | Lécithine de soja 10,1 | Esters d'acide citrique 3,5 | Alpha-tocophérol 0,9 | Eau 3,9 | - | AGPI 78,1 | Vit. E 3,5 |
| **4** | Lécithine de soja 13,5 | - | - | Eau 5,8 | Maltodex-trines 0,3 | AGPI 76,9 | Vit. E 3,5 |
| **5** | Lécithine de soja 13 | - | Alpha- tocophérol 0,8 | Eau 8,12 | Amidon réticulé 0,08 | AGPI 74,6 | Vit. E 3,4 |
| **6** | Lécithine de soja 13,1 | - | Alpha-tocophérol 0,8 | Eau 7,49 | Caséinate de Na 0,11 | AGPI 75,1 | Vit. E 3,4 E |
| **7** | Lécithine de soja 16,4 | - | - | Eau 7,19 | Caséinate de Na 0,11 | AGPI 73,0 | Vit. E 3,3 |
| **8** | Lécithine de soja 13,1 | - | Alpha-tocophérol 0,8 | Eau 7,59 | Gomme xanthane 0,01 | AGPI 75,1 | Vit. E 3,4 |
| **9** | Lécithine de soja 13,1 | - | Alpha-tocophérol 0,8 | Eau 7,48 | Caséinate de Na 0,11 Gomme xanthane 0,008 | AGPI 75,1 | Vit. E 3,4 |
| **10** | Lécithine soja 16,4 | - | - | Eau 7,18 | Caséinate de Na 0,11 Gomme xanthane 0,007 | AGPI 73,0 | Vit. E 3,3 |

### Exemple 2 : margarine incorporant des vésicules dont la phase hydrophobe est constituée d'une huile de poisson riche en oméga 3/6.

Des vésicules répondant à la formulation 9 du tableau I, mais dont la phase hydrophobe est constituée d'une huile de poisson, ont été préparées selon le même procédé que dans l'exemple 1.

Indépendamment, des margarines (émulsions eau dans huile) ont été fabriquées selon un procédé classique connu de l'homme de l'art comportant :
- la préparation d'une phase grasse à chaud,
- la préparation d'une phase aqueuse,
- l'addition lente de la phase aqueuse dans la phase grasse sous agitation forte pour créer l'émulsion.

Ces étapes sont classiquement suivies d'une étape de pasteurisation, puis de la texturation de l'émulsion par refroidissement malaxage.

La margarine est ensuite conditionnée par exemple en barquettes et stockée au froid.

Dans une première série de margarines, les vésicules fabriquées en parallèle ont été introduites (en une quantité correspondant à 11,5%, en pourcentage massique par rapport à la masse totale de la phase grasse) après l'obtention de l'émulsion (avant l'étape de pasteurisation). Dans une autre série, elles l'ont été après la texturation (avant le conditionnement).

Au final, la phase grasse des margarines représente 59% du poids total de la composition et la phase aqueuse représente donc 41 % du poids total de la composition.

La composition de la phase grasse est indiquée dans le tableau II.

**Tableau II : composition de la phase grasse de la margarine**

| **constituants** | **% phase grasse** |
|---|---|
| Huile de colza | 52,7 |
| Huile de palmiste | 14,4 |
| Huile de tournesol oléique | 8,8 |
| Huile de palme | 6,4 |
| MGLA | 5,0 |
| Structures uni- ou multivésiculaires avec huile de poisson | 11,5 |
| Additifs liposolubles | 1,2 |

Des tests de dégustation des deux séries de margarine ont été réalisés. Dans les deux cas, les produits ont un goût relativement neutre. L'incorporation « après texturation » s'est révélée encore plus efficace que celle « après émulsion » pour éviter l'apparition d'odeur et de goût désagréables de l'huile de poisson.

Dans une variante, on peut remplacer l'huile de poisson des vésicules par une huile de tournesol, et on incorpore en plus un arôme de noisette dans la phase hydrophile de la bicouche (3) des vésicules. On obtient ainsi une margarine ayant un goût de noisette.

Exemple 3 : huile corporelle incorporant des vésicules dont la phase hydrophobe est constituée du mélange d'un composé neutre hydrophobe et d'un composé d'intérêt hydrophobe qui est une huile essentielle, et dont la phase hydrophile contenue dans la bicouche comprend un composé d'intérêt hydrophile qui est un polyol.

L'huile corporelle est fabriquée selon les méthodes usuelles. Des vésicules dont la phase hydrophobe est constituée d'un mélange comprenant pour moitié un composé neutre hydrophobe, à savoir un mélange d'huile d'arachide et d'huile de palme en proportion égale, et pour autre moitié un composé d'intérêt hydrophobe qui est une huile essentielle de citron, et dont la phase hydrophile contenue dans la bicouche (3) comprend un composé d'intérêt hydrophile qui est un polyol, sont fabriquées selon le procédé de la présente invention, puis introduites dans l'huile corporelle qui est un produit constitué seulement d'une phase grasse (4).

## Revendications

1. Vésicule (1) à caractère hydrophobe et incorporable dans une phase grasse **caractérisée en ce qu'**elle comprend :
- une cavité interne hydrophobe (2) huileuse constituée soit d'au moins un composé neutre hydrophobe, soit au moins d'un composé d'intérêt hydrophobe, soit à la fois d'au moins un composé neutre hydrophobe et d'au moins d'un composé d'intérêt hydrophobe et
- au moins une bicouche (3) constituée de deux couches de lipides amphiphiles (6) déterminant entre elles un espace interstitiel hydrophile (7), et
- le composé neutre hydrophobe est une huile neutre choisie parmi les huiles d'arachide *(Arachis hypogea),* d'adjouaba *(Haematostaphis barteri),* d'avocat (Persea americana), de babassu *(Orbignya oleifera),* de beurre de karité *(Vitellaria paradoxa),* de beurre de cacao *(Theobroma cacao*), de chufa *(Cyperus esculentus),* de crambe *(Crambe abyssinica),* de cuphea *(Cuphea tolucana, Cuphea wrightii, Cuphea laminuligena, Cuphea ilavea* ou *Cuphea leptopoda),* d'érable *(Acer saccharum* ou *Acer saccharinum),* de Gokhru *(Tribulus terrestris)*, de noisette *(Corylus avellana),* de noix de coco *(Cocus nucifera),* de noix de Macadamia *(Macadamia tetraphylla),* de noix du Cambodge *(Irvingia malayana),* de noix pili *(Canarium ovatum),* de noyau d'abricot *(Prunus armeniaca),* d'olive *(Olea europaea),* de palme *(Elaeis guineensis),* de palmiste *(Elaeis guineensis),* de pépins de mangue *(Mangifer indica),* de pistache *(Pistacia atlantica),* ou des fractions de ces huiles (comme par exemple les oléines et stéarines de palme ou de palmiste), ladite huile étant seule ou en mélange,
- le composé d'intérêt hydrophobe est choisi parmi les principes actifs ou ingrédients pharmaceutiques et/ou cosmétiques hydrophobes ou les principes actifs ou ingrédients alimentaires hydrophobes ou,
- est une huile choisie parmi les huiles essentielles, les huiles polyinsaturées marines ou les huiles polyinsaturées végétales, seules ou en mélange.

2. Vésicule (1) selon la revendication 1 **caractérisée en ce que** l'huile polyinsaturée marine est choisie parmi les huiles de poisson.

3. Vésicule (1) selon la revendication 1 ou 2 **caractérisée en ce que** l'huile essentielle est choisie parmi l'huile essentielle de citron, de lavande, d'eucalyptus, de camphre, de sauge, de thym, de girofle.

4. Vésicule (1) selon l'une quelconque des revendications 1 à 3 **caractérisée en ce que** l'huile polyinsaturée végétale est une huile riche en acides gras oméga 3 ou en acides gras oméga 6.

5. Vésicule (1) selon l'une quelconque des revendications 1 à 4 **caractérisée en ce que** lorsque le nombre de bicouche (3) de lipides amphiphiles (6) est supérieur ou égal à 2, lesdites bicouches (3) sont concentriques.

6. Vésicule (1) selon l'une quelconque des revendications 1 à 4 **caractérisée en ce que** lorsque le nombre de bicouche (3) de lipides amphiphiles (6) est supérieur ou égal à 3, lesdites bicouches (3) sont juxtaposées.

7. Vésicule (1) selon l'une quelconque des revendications 1 à 4 caractérisée en ce lorsque le nombre de bicouche (3) de lipides amphiphiles (6) est supérieur ou égal à 4, lesdites bicouches (3) sont concentriques et juxtaposées.

8. Vésicule (1) selon l'une quelconque des revendications 1 à 7 caractérisée ce que les lipides amphiphiles (6) ont leur partie hydrophile orientée tête-à-tête vers l'intérieur de cette bicouche (3), délimitant ainsi l'espace interstitiel hydrophile (7).

9. Vésicule (1) selon l'une quelconque des revendications 1 à 8, **caractérisée en ce que** les lipides amphiphiles (6) de la bicouche (3) sont des phospholipides.

10. Vésicule (1) selon la revendication 9 **caractérisée en ce que** les lipides amphiphiles (6) de la bicouche (3) sont des lécithines du soja.

11. Vésicule (1) selon l'une quelconque des revendications 1 à 10 **caractérisée en ce que** la bicouche (3) comprend en outre au moins un émulsifiant, tel que ceux choisis parmi le groupe des monoglycérides ou des diglycérides d'acides gras.

12. Vésicule (1) selon l'une quelconque des revendications 1 à 11 **caractérisée en ce que** la bicouche (3) comprend en outre au moins un composé stabilisateur, tel que ceux choisis parmi les composés de type tocophérol comme l'alpha-tocophérol, ou de type stérol comme le cholestérol.

13. Vésicule (1) selon l'une quelconque des revendications 1 à 12 **caractérisée en ce que** l'espace interstitiel hydrophile (7) contenu dans la bicouche (3) est constitué d'eau.

14. Vésicule (1) selon l'une quelconque des revendications 1 à 13 **caractérisée en ce que** l'espace interstitiel hydrophile (7) déterminé par la bicouche (3) comprend en outre au moins un agent faisant barrière à l'oxygène.

15. Vésicule (1) selon la revendication 14 **caractérisée en ce que** le au moins un agent faisant barrière à l'oxygène est choisi parmi les gels d'hydrocolloïdes peu hydratés, la cire d'abeille, l'isolat de protéines de lactosérum, l'isolat de protéines de soja, la β-lactoglobuline, la caséine, les sels de caséine tels que le caséinate de sodium, le sorbitol, le glycérol, pris seul ou en mélange.

16. Vésicule (1) selon l'une quelconque des revendications 1 à 15 **caractérisée en ce que** l'espace interstitiel hydrophile (7) déterminé par la bicouche (3) comprend en outre au moins un agent capable d'augmenter la rigidité de ladite bicouche (3) et/ou de diminuer la disponibilité de la phase hydrophile.

17. Vésicule (1) selon la revendication 16 **caractérisée en ce que** le au moins un agent capable d'augmenter la rigidité de ladite bicouche (3) et/ou de diminuer la disponibilité de la phase hydrophile est choisi parmi les hydrocolloïdes tels que l'amidon natif (de blé, de riz, de maïs, de paprika, de pomme de terre), l'amidon réticulé, l'amidon prégélatinisé, l'amylopectine, ou les produits de dégradation de l'amidon comme les maltodextrines de différents degrés d'hydrolyse, les gommes telles que les gommes d'acacia, les gommes guar, les gommes xanthane, les gommes adragante, les gommes karaya, les gommes tara, les gommes gellane, le sorbitol, le sirop de sorbitol, le mannitol, le glycérol, le glucomannane, le stéarate de polyoxyéthylène (8), le stéarate de polyoxyéthylène (40), le monolaurate de polyoxyéthylène sorbitane (polysorbate 20), le monooléate de polyoxyéthylène de sorbitane (polysorbate 80), le monopalmitate de polyoxyéthylène sorbitane (polysorbate 40), le monostéarate de polyoxyéthylène sorbitane (polysorbate 60), le tristéarate de polyoxyéthylène sorbitane (polysorbate 65), la pectine, la pectine amidée, les phosphatides d'ammonium, l'acétate isobutyrate de saccharose, les esters glycériques de résine de bois, l'acide alginique, les alginates de sodium, de potassium, d'ammonium, de calcium ou de propane-1,2-diol, l'agar-agar, les carraghénanes, les algues euchema transformées ou la farine de graines de caroube, pris seuls ou en mélange.

18. Vésicule (1) selon l'une quelconque des revendications 1 à 17 **caractérisée en ce que** l'espace interstitiel hydrophile (7) déterminé par la bicouche (3) comprend au moins un composé d'intérêt hydrophile.

19. Vésicule (1) selon la revendication 18 **caractérisée en ce que** le composé d'intérêt hydrophile est choisi parmi les principes actifs ou ingrédients pharmaceutiques et/ou cosmétiques hydrophiles ou les principes actifs ou ingrédients alimentaires hydrophiles.

20. Vésicule (1) selon la revendication 19 **caractérisée en ce que** le composé d'intérêt hydrophile est choisi parmi les antitumoraux (adriamycine, actimycine, mitomycine, 1 β-arabinofuranosyl cytosine, cisplatine), les antiviraux (interféron), les aminoglucosides (gentamicine), les antibiotiques (β-lactam, sulbenicilline, céfotiame, cefmenoxime), les hormones peptidiques (TRH, leuprolide, insuline), les agents immunopotentialisateurs (muramyldipeptide, muramyltripeptide), les protéines (immunoglobulines, toxines), les agents anti-allergiques, antimycotiques, cytostatiques ou de diagnostic, les anxiolytiques, les contraceptifs, les sédatifs, les sels minéraux (calcium, chlore, magnésium, phosphore, potassium, sodium, soufre), les oligo-éléments (aluminium, brome, cuivre, cobalt, fer, fluor, manganèse, molybdène, iode, sélénium, silicium, vanadium, zinc), les glucides (comme le glucose, le galactose, le mannose, le maltose, le maltotriose), les acides aminés (alanine, arginine, asparagine, acide aspartique, cystéine, acide glutamique, glutamine, glycine, histidine, isoleucine, leucine, lysine, méthionine, phénylalanine, proline, sérine, thréonine, tryptophane, tyrosine, valine), les peptides, les protéines, les vitamines hydrosolubles, les arômes (de citron, de vanille, de fraise, de café, de noisette, de framboise...), les polyols.

21. Vésicule (1) selon l'une des revendications 1 à 20 **caractérisée en ce que** le composé d'intérêt hydrophobe est choisi parmi le paracétamol, le pindolol, le probucol.

22. Vésicule (1) selon l'une des revendications 1 à 20 **caractérisée en ce que** le composé d'intérêt hydrophobe est choisi parmi les antioxydants tels que l'extrait de romarin, de thé vert, un mélange de tocophérols, les acides gras libres tels que l'EPA, le DHA, l'acide arachidonique, les acides gras essentiels tels que l'acide linoléique, l'acide alpha-linolénique, les stérols végétaux, les stanols végétaux, les polycosanols, les vitamines liposolubles telles que la vitamine A, D, E, K, les colorants tels que les caroténoïdes, les flavonoïdes, les composés aromatiques tel que le diacétyle, les huiles alimentaires aromatiques (huiles vierges de noix, d'olive, de noisette).

23. Vésicule (1) selon l'une quelconque des revendications 1 à 22 **caractérisée en ce que** la cavité interne hydrophobe comprend en outre au moins un antioxydant.

24. Vésicule (1) selon la revendication 23 **caractérisée en ce que** le au moins un antioxydant est choisi parmi les tocophérols sous la forme d'un mélange naturel, ou de alpha-tocophérol de synthèse, ou de gamma-tocophérol de synthèse, ou de delta-tocophérol de synthèse, le palmitate d'ascorbyle, le stéarate d'ascorbyle, le gallate de propyle, le gallate d'octyle, le gallate dodécyle, le BHA (buthylhydroxyanisol), le BHT (buthylhydroxytoluène), l'EDTA (éthylène diamine tétra acétate), l'extrait naturel de romarin, l'extrait de thé vert, les polyphénols, pris seul ou en mélange.

25. Vésicule (1) selon l'une quelconque des revendications 1 à 24 **caractérisée en ce que** en pourcentage massique par rapport à la masse totale de la vésicule (1) :
- le pourcentage massique de la cavité interne hydrophobe (2) est compris entre 70 et 89%,
- le pourcentage massique de la bicouche (3) de lipides amphiphiles (6) est compris entre 10 et 22,5%,
- le pourcentage massique de l'espace interstitiel hydrophile (7) contenu dans la bicouche (3) est compris entre 3,8 et 8,5%.

26. Vésicule (1) selon la revendication 25 **caractérisée en ce que** en pourcentage massique par rapport à la masse totale de la vésicule (1), la cavité interne hydrophobe (2) est constituée :
- d'au moins un composé neutre hydrophobe et/ou d'au moins un composé d'intérêt hydrophobe dont le pourcentage massique est compris entre 70 et 89%,
et comprend en outre :
- au moins un antioxydant, dont le pourcentage massique est compris entre 0 et 4%.

27. Vésicule (1) selon la revendication 25 **caractérisée en ce que** en pourcentage massique par rapport à la masse totale de la vésicule (1), la bicouche (3) de lipides amphiphiles (6) est constituée :
- de lipides amphiphiles (6), dont le pourcentage est compris entre 10 et 22,5%, et comprend en outre :
- au moins un émulsifiant, dont le pourcentage massique est compris entre 0 et 4,5%,
et comprend en outre :
- au moins un stabilisateur, dont le pourcentage massique est compris entre 0 à 1 %.

28. Vésicule (1) selon la revendication 25 **caractérisée en ce que** en pourcentage massique par rapport à la masse totale de la vésicule (1), l'espace interstitiel hydrophile (7) est constitué :
- d'eau, dont le pourcentage massique est compris entre 3,8 et 8,5%,
et comprend en outre
- au moins un agent faisant barrière à l'oxygène, dont le pourcentage massique est compris entre 0 et 0,2%, et/ou
- au moins un agent capable d'augmenter la rigidité de la bicouche lipidique et/ou de diminuer la disponibilité de la phase hydrophile, dont le pourcentage massique est compris entre 0 et 2,135%.

29. Vésicule (1) selon la revendication 28 **caractérisée en ce que** en pourcentage massique par rapport à la masse totale de la vésicule (1), l'espace interstitiel hydrophile (7) comprend
- de l'amidon natif ou réticulé, dont le pourcentage massique est compris entre 0 et 1,5%,
- et/ou des maltodextrines, dont le pourcentage massique est compris entre 0 et 0,3%,
- et/ou de gomme d'acacia, dont le pourcentage massique est compris entre 0 et 0,3%,
- et/ou de la gomme guar, dont le pourcentage est compris massique entre 0 et 0,02%,
- et/ou de la gomme de xanthane, dont le pourcentage massique est compris entre 0 et 0,015%.

30. Vésicule (1) selon l'une quelconque des revendications 25 à 29 **caractérisée en ce que** en pourcentage massique par rapport à la masse totale de la vésicule (1):
- la cavité interne hydrophobe (2) est constituée d'au moins un composé neutre hydrophobe et/ou d'au moins un composé d'intérêt hydrophobe, dont le pourcentage massique est de 75%, et d'au moins un antioxydant dont le pourcentage massique est de 3,5%,
- la bicouche (3) de lipides amphiphiles (6) est constituée de lipides amphiphiles dont le pourcentage massique est compris entre 13 et 16%, et comprend au moins un émulsifiant dont le pourcentage massique est de 3,5%, et au moins un stabilisateur dont le pourcentage massique est de 0,8%,
- l'espace interstitiel hydrophile (7) est constitué d'eau, dont le pourcentage massique est de 4%, il comprend au moins un agent faisant barrière à l'oxygène dont le pourcentage massique est de 0,11%, et il comprend au moins un agent capable d'augmenter la rigidité de la bicouche lipidique et/ou de diminuer la disponibilité de la phase hydrophile dont le pourcentage massique est de 0,705%.

31. Vésicule (1) selon la revendication 30 **caractérisée en ce que** en pourcentage massique par rapport à la masse totale de la vésicule (1), l'espace interstitiel hydrophile (7) comprend :
- de l'amidon natif ou réticulé, dont le pourcentage massique est de 0,08%,
- des maltodextrines, dont le pourcentage massique est de 0,3%,
- de la gomme acacia, dont le pourcentage massique est de 0,3%,
- de la gomme guar, dont le pourcentage massique est de 0,015%,
- de la gomme xanthane, dont le pourcentage massique est de 0,01 %.

32. Phase grasse (4) d'un produit **caractérisée en ce qu'**elle comprend au moins une vésicule (1) telle que définie dans l'une des revendications 1 à 31.

33. Produits cosmétiques ou pharmaceutiques **caractérisés en ce qu'**ils comprennent au moins une vésicule (1) telle que définie dans les revendications 1 à 31.

34. Produits alimentaires **caractérisés en ce qu'**ils comprennent au moins une vésicule (1) telle que définie dans les revendications 1 à 31.

35. Produits cosmétiques ou pharmaceutiques selon la revendication 33 **caractérisés en ce qu'**ils comprennent dans leur phase grasse (4) au moins une vésicule (1) telle que définie dans les revendications 1 à 31.

36. Produits alimentaires selon la revendication 34 **caractérisés en ce qu'**ils comprennent dans leur phase grasse (4) au moins une vésicule (1) telle que définie dans les revendications 1 à 31.

37. Procédé de préparation de vésicules (1) telle que définies selon l'une quelconque des revendications 1 à 31 comprenant les étapes suivantes :
- étape A : étalement des lipides amphiphiles (6),
- étape B : addition d'une phase hydrophile dont sera constitué l'espace interstitiel hydrophile (7),
- étape C : pose pour obtenir des bicouches lipidiques (3),
- étape D : élimination de l'excès de la phase hydrophile (7) pour obtenir des films phospholipidiques hydratés (5),
- étape E : addition d'une phase hydrophobe dont sera constitué la cavité interne hydrophobe (2),
- étape F : agitation forte.

38. Procédé selon la revendication 37 **caractérisé en ce que** :
- dans l'étape A, l'étalement des lipides amphiphiles (6) est effectué sur la paroi d'un réacteur en rotation ou sur une surface plane,
- dans l'étape B, la phase hydrophile est ajoutée dans le réacteur, ou elle atomisée sur la surface plane ou la surface plane est immergée dans un bain de phase hydrophile,
- dans l'étape E, la phase hydrophobe est ajoutée dans le réacteur ou placée dans un récipient puis ajoutée par-dessus les films phospholipidiques hydratés (5).

39. Procédé selon l'une ou l'autre des revendications 37 et 38 **caractérisé en ce que** dans l'étape E la phase hydrophobe est constituée en outre d'au moins un composé d'intérêt hydrophobe

40. Procédé selon la revendication 39 **caractérisé en ce que** le composé d'intérêt hydrophobe est une huile de poisson.

41. Procédé selon l'une quelconque des revendications 37 à 40 **caractérisé en ce qu'**il comprend une étape supplémentaire :
- étape G : décantation et récupération des vésicules.

42. Procédé selon la revendication 41 **caractérisée en ce qu'**il comprend une étape supplémentaire :
- étape H : introduction des vésicules dans la phase grasse (4) d'un produit.

43. Utilisation de vésicules (1) telles que définies selon l'une quelconque des revendications 1 à 31 comme système transporteur-délivreur d'au moins un composé d'intérêt hydrophobe et le cas échéant d'au moins un composé d'intérêt hydrophile.

44. Utilisation selon la revendication 43 **caractérisée en ce que** le composé d'intérêt hydrophile est choisi parmi les principes actifs ou ingrédients pharmaceutiques et/ou cosmétiques hydrophiles ou les principes actifs ou ingrédients alimentaires hydrophiles, et il se trouve dans l'espace interstitiel hydrophile (7) contenu dans la bicouche (3) de lipides amphiphiles (6).

45. Utilisation selon la revendication 43 **caractérisée en ce que** le composé d'intérêt hydrophobe est choisi parmi les principes actifs ou ingrédients pharmaceutiques et/ou cosmétiques hydrophobes ou les principes actifs ou ingrédients alimentaires hydrophobes, et il se trouve dans la cavité interne hydrophobe (2).

46. Utilisation selon la revendication 45 **caractérisée en ce que** le composé d'intérêt hydrophobe est une huile choisie parmi les huiles essentielles, les huiles polyinsaturées marines ou les huiles polyinsaturées végétales.

47. Utilisation selon la revendication 46 **caractérisée en ce que** le composé d'intérêt hydrophobe est une huile de poisson.

48. Utilisation de vésicules (1) telles que définies selon l'une quelconque des revendications 1 à 31 pour la protection contre l'hydrolyse et/ou l'oxydation d'au moins un composé d'intérêt, notamment hydrophobe.

49. Utilisation selon la revendication 48 **caractérisée en ce que** le composé d'intérêt est une huile choisie parmi les huiles essentielles, les huiles polyinsaturées marines ou les huiles polyinsaturées végétales.

50. Utilisation selon la revendication 49 **caractérisée en ce que** le composé d'intérêt est une huile de poisson.

51. Utilisation de vésicules (1) telles que définies selon l'une quelconque des revendications 1 à 31 pour enrichir des produits constitués d'une phase grasse (4) en au moins un composé d'intérêt hydrophobe présentant un fort taux d'acides gras insaturés et/ou d'oméga 3/6.

52. Utilisation selon la revendication 51 **caractérisée en ce que** le composé d'intérêt hydrophobe est une huile de poisson.

53. Utilisation dans des produits alimentaires, cosmétiques ou pharmaceutiques de vésicules (1) telles que définies selon l'une quelconque des revendications 1 à 31, comprenant dans la cavité interne hydrophobe (2) un composé d'intérêt hydrophobe qui est une huile, pour empêcher le développement d'odeurs et/ou de goûts désagréables dégagés par ladite huile.

54. Utilisation selon la revendication 53 **caractérisée en ce que** l'huile est une huile choisie parmi les huiles essentielles, les huiles polyinsaturées marines ou les huiles polyinsaturées végétales.

55. Utilisation selon la revendication 54 **caractérisée en ce que** l'huile est une huile de poisson.

## Patentansprüche

1. Vesikel (1) mit hydrophobem Charakter und in eine Fettphase einarbeitbar, **dadurch gekennzeichnet, dass** es folgendes aufweist:
- einen inneren hydrophoben öligen Hohlraum (2), der aus mindestens einer neutralen hydrophoben Verbindung oder mindestens einer hydrophoben Verbindung von Interesse oder gleichzeitig mindestens einer neutralen hydrophoben Verbindung und mindestens einer hydrophoben Verbindung von Interesse gebildet wird, und
- mindestens eine Doppelschicht (3), die aus zwei Schichten aus amphiphilen Lipiden (6) gebildet wird, die zwischen ihnen einen hydrophilen interstitiellen Raum (7) definiert, und dass
- die neutrale hydrophobe Verbindung ein neutrales Öl ist, das ausgewählt ist aus den Ölen von Erdnuss (Arachis hypogea), von Adiouaba (Haematostaphis barteri), von Avocado (Persea americana), von Babassu (Orbignya oleifera), Sheabutter (Vitellaria paradoxa), Kakaobutter (Theobroma cacao), von Erdmandel (Cyperus esculentus), von Meerkohl (Crambe abyssinica), von Cuphea (Cuphea tolucana, Cuphea wrightii, Cuphea laminuligena, Cuphea ilavea oder Cuphea leptopoda), von Ahorn (Acer saccharum oder Acer saccharinum), von Gokhru (Tribulus terrestris), von Haselnuss (Corylus avellana), von Kokosnuss (Cocus nucifera), von Macadamia-Nuss (Macadamia tetraphylla), von der Cambodge-Nuss (Irvingia malayana), von Pili-Nuss (Canarium ovatum), von Aprikosenkern (Prunus armeniaca), von Olive (Olea europaea), Palmöl (Elaeis guineensis), Palmkernöl (Elaeis guineensis), von Mangokernen (Mangifer indica), von Pistazie (Pistacia atlantica) oder Fraktionen dieser Öle (zum Beispiel Oleine und Stearine von Palm- oder Palmkernöl), wobei das Öl einzeln oder als Gemisch vorliegt,
- die hydrophobe Verbindung von Interesse ausgewählt ist aus hydrophoben pharmazeutischen und/oder kosmetischen Wirkstoffen oder Ingredienzien oder hydrophoben alimentären Wirkstoffen oder Ingredienzien ausgewählt ist, oder
- ein Öl, ausgewählt aus den ätherischen Ölen, den marinen mehrfach ungesättigten Ölen oder den pflanzlichen mehrfach ungesättigten Ölen, allein oder als Gemisch, ist.

2. Vesikel (1) gemäß Anspruch 1,
**dadurch gekennzeichnet,**
**dass** das marine mehrfach ungesättigte Öl aus Fischölen ausgewählt ist.

3. Vesikel (1) gemäß Anspruch 1 oder 2,
**dadurch gekennzeichnet,**
**dass** das ätherische Öl aus dem ätherischen Öl von Zitrone, von Lavendel, von Eukalyptus, von Kampfer, von Salbei, von Thymian, von Furunkel ausgewählt ist.

4. Vesikel (1) gemäß einem der Ansprüche 1 bis 3,
**dadurch gekennzeichnet,**
**dass** das pflanzliche mehrfach ungesättigte Öl ein Öl ist, das reich an Omega-3-Fettsäuren oder Omega-6-Fettsäuren ist.

5. Vesikel (1) gemäß einem der Ansprüche 1 bis 4,
**dadurch gekennzeichnet,**
**dass**, wenn die Zahl der Doppelschichten (3) aus amphiphilen Lipiden (6) größer als oder gleich 2 ist, die Doppelschichten (3) konzentrisch sind.

6. Vesikel (1) gemäß einem der Ansprüche 1 bis 4,
**dadurch gekennzeichnet,**
**dass**, wenn die Zahl der Doppelschichen (3) aus amphiphilen Lipiden (6) größer als oder gleich 3 ist, die Doppelschichten (3) nebeneinander liegend sind.

7. Vesikel (1) gemäß einem der Ansprüche 1 bis 4,
**dadurch gekennzeichnet,**
**dass**, wenn die Zahl der Doppelschichten (3) aus amphiphilen Lipiden (6) größer als oder gleich 4 ist, die Doppelschichten (3) konzentrisch und nebeneinander liegend sind.

8. Vesikel (1) gemäß einem der Ansprüche 1 bis 7,
**dadurch gekennzeichnet,**
**dass** die amphiphilen Lipide (6) ihren hydrophilen Teil Kopf-an-Kopf zum Inneren dieser Doppelschicht (3) orientiert haben, was den hydrophilen interstitiellen Raum (7) begrenzt.

9. Vesikel (1) gemäß einem der Ansprüche 1 bis 8,
**dadurch gekennzeichnet,**
**dass** die amphiphilen Lipide (6) der Doppelschicht (3) Phospholipide sind.

10. Vesikel (1) gemäß Anspruch 9,
**dadurch gekennzeichnet,**
**dass** die amphiphilen Lipide (6) der Doppelschicht (3) Sojalecithine sind.

11. Vesikel (1) gemäß einem der Ansprüche 1 bis 10,
**dadurch gekennzeichnet,**
**dass** die Doppelschicht (3) außerdem mindestens einen Emulgator wie die, ausgewählt aus der Gruppe der Fettsäure-monoglyceride oder -diglyceride, umfasst.

12. Vesikel (1) gemäß einem der Ansprüche 1 bis 11,
**dadurch gekennzeichnet,**
**dass** die Doppelschicht (3) außerdem mindestens eine stabilisierende Verbindung wie die, ausgewählt aus den Verbindungen des Tocopherol-Typs wie alpha-Tocopherol oder des Sterol-Typs wie Cholesterin, umfasst.

13. Vesikel (1) gemäß einem der Ansprüche 1 bis 12,
**dadurch gekennzeichnet,**
**dass** der hydrophile interstitielle Raum (7), der in der Doppelschicht (3) enthalten ist, aus Wasser besteht.

14. Vesikel (1) gemäß einem der Ansprüche 1 bis 13,
**dadurch gekennzeichnet,**
**dass** der hydrophile interstitielle Raum (7), der durch die Doppelschicht (3) definiert wird, außerdem mindestens ein Mittel umfasst, das eine Barriere gegen Sauerstoff bildet.

15. Vesikel (1) gemäß Anspruch 14,
**dadurch gekennzeichnet,**
**dass** das mindestens eine Mittel, das eine Barriere gegen Sauerstoff bildet, ausgewählt ist aus den wenig hydratisierten Hydrokolloiden, Bienenwachs, Isolat von Molkeproteinen, Isolat von Sojaproteinen, β-Lactoglobulin, Casein, Caseinsalzen wie Natriumcaseinat, Sorbit, Glycerin, allein oder als Gemisch.

16. Vesikel (1) gemäß einem der Ansprüche 1 bis 15,
**dadurch gekennzeichnet,**
**dass** der hydrophile interstitielle Raum (7), der durch die Doppelschicht (3) definiert wird, außerdem mindestens ein Mittel umfasst, das fähig ist, die Steifigkeit der Doppelschicht (3) zu erhöhen und/oder die Verfügbarkeit der hydrophilen Phase zu vermindern.

17. Vesikel (1) gemäß Anspruch 16,
**dadurch gekennzeichnet,**
**dass** das mindestens eine Mittel, das geeignet ist, die Steifigkeit der Doppelschicht (3) zu erhöhen und/oder die Verfügbarkeit der hydrophilen Phase zu vermindern, ausgewählt ist aus Hydrokolloiden wie native Stärke (von Weizen, Reis, Mais, Paprika, Kartoffel), vernetzter Stärke, vorgelatinierter Stärke, Amylopektin oder den Abbauprodukten der Stärke wie Maltodextrine unterschiedlichen Hydrolysegrads, Gummen wie Akazien-Gummen, Guar-Gummen, Xanthan-Gummen, Tragant-Gummen, Karaya-Gummen, Tara-Gummen, Gellan-Gummen, Sorbit, Sorbitsirup, Mannit, Glycerin, Glucomannan, Polyoxyethylen(8)-stearat, Polyoxyethylen(40)-stearat, Polyoxyethylensorbitan-monolaurat (Polysorbat 20), Polyoxyethylensorbitanmonooleat (Polysorbat 80), Polyoxyethylensorbitan-monopalmitat (Polysorbat 40), Polyoxyethylensorbitan-monostearat (Polysorbat 60), Polyoxyethylensorbitan-tristearat (Polysorbat 65), Pektin, amidiertem Pektin, Ammoniumphosphatide, Saccharoseacetat-isobutyrat, Glycerinester von Holzharz, Alginsäure, Natrium-, Kalium-, Ammonium-, Calcium- oder Propan-1,2-diol-alginaten, Agar-Agar, Carrageenanen, transformierten Euchema-Algen oder Johannisbrotkernmehl, allein oder als Gemisch genommen.

18. Vesikel (1) gemäß einem der Ansprüche 1 bis 17,
**dadurch gekennzeichnet,**
**dass** der hydrophile interstitielle Raum (7), der durch die Doppelschicht (3) definiert wird, mindestens eine hydrophile Verbindung von Interesse umfasst.

19. Vesikel (1) gemäß Anspruch 18,
**dadurch gekennzeichnet,**
**dass** die hydrophile Verbindung von Interesse ausgewählt ist aus hydrophilen pharmazeutischen und/oder kosmetischen Wirkstoffen oder Ingredienzien oder hydrophilen alimentären Wirkstoffen oder Ingredienzien.

20. Vesikel (1) gemäß Anspruch 19,
**dadurch gekennzeichnet,**
**dass** die hydrophile Verbindung von Interesse ausgewählt ist aus Antitumormitteln (Adriamycin, Actimycin, Mitomycin, 1 β-Arabinofuranosylcytosin, Cisplatin), antiviralen Mitteln (Interferon), Aminoglucosiden (Gentamicin), Antibiotika (β-Lactam, Sulbenicillin, Cefotiam, Cefmenoxim), peptidischen Hormonen (TRH, Leuprolid, Insulin), Immunpotenzierungsmitteln (Muramyldipeptid, Muramyltripeptid), Proteinen (Immunglobulinen, Toxinen), Antiallergika, Antimycotika, Cytostatika oder Diagnostika, Anxiolytika, Kontrazeptiva, Sedativa, Mineralsalzen (Calcium, Chlor, Magnesium, Phosphor, Kalium, Natrium, Schwefel), Spurenelementen (Aluminium, Brom, Kupfer, Kobalt, Eisen, Fluor, Mangan, Molybdän, Iod, Selen, Silicium, Vanadium, Zink), Kohlenhydraten (wie Glucose, Galactose, Mannose, Maltose, Maltotriose), Aminosäuren (Alanin, Arginin, Asparagin, Asparaginsäure, Cystein, Glutaminsäure, Glutamin, Glycin, Histidin, Isoleucin, Leucin, Lysin, Methionin, Phenylalanin, Prolin, Serin, Threonin, Tryptophan, Tyrosin, Valin), Peptiden, Proteinen, wasserlöslichen Vitaminen, Aromastoffen (Zitrone, Vanille, Erdbeere, Kaffee, Nuss, Himbeere, ...), Polyolen.

21. Vesikel (1) gemäß einem der Ansprüche 1 bis 20,
**dadurch gekennzeichnet,**
**dass** die hydrophobe Verbindung von Interesse aus Paracetamol, Pindolol, Probucol ausgewählt ist.

22. Vesikel (1) gemäß einem der Ansprüche 1 bis 20,
**dadurch gekennzeichnet,**
**dass** die hydrophobe Verbindung von Interesse aus Antioxidanzien wie dem Extrakt von Rosmarin, von grünem Tee, einem Tocopherol-Gemisch, freien Fettsäuren wie EPA, DHA, Arachidonsäure, essentiellen Fettsäuren wie Linolsäure, alpha-Linolensäure, pflanzlichen Sterolen, pflanzlichen Stanolen, Polycosanolen, fettlöslichen Vitaminen wie Vitamin A, D, E, K, Färbemitteln wie Carotenoide, Flavonoide, aromatischen Verbindungen wie Diacetyl, aromatischen Speiseölen (native Walnuss-, Oliven-, Haselnussöle).

23. Vesikel (1) gemäß einem der Ansprüche 1 bis 22,
**dadurch gekennzeichnet,**
**dass** der hydrophobe innere Hohlraum außerdem mindestens ein Antioxidans umfasst.

24. Vesikel (1) gemäß Anspruch 23,
**dadurch gekennzeichnet,**
**dass** das mindestens eine Antioxidans ausgewählt ist aus den Tocopherolen in Form eines natürlichen Gemisches oder in Form von synthetischem alpha-Tocopherol oder synthetischem gamma-Tocopherol oder synthetischem delta-Tocopherol, Ascorbylpalmitat, Ascorbylstearat, Propylgallat, Octylgallat, Dodecylgallat, BHA (Butylhydroxyanisol), BHT (Butylhydroxytoluol), EDTA (Ethylendiamintetraacetat), dem natürlichen Extrakt von Rosmarin, dem Extrakt von grünem Tee, Polyphenolen, einzeln oder als Gemisch genommen.

25. Vesikel (1) gemäß einem der Ansprüche 1 bis 24,
**dadurch gekennzeichnet,**
**dass** als Massenprozentanteil, bezogen auf die Gesamtmasse des Vesikels (1),
- der Massenprozentanteil des hydrophoben inneren Hohlraums (2) zwischen 70 % und 89 % liegt,
- der Massenprozentanteil der Doppelschicht (3) aus amphiphilen Lipiden (6) zwischen 10 % und 22,5 % liegt,
- der Massenprozentanteil des hydrophilen interstitiellen Raums (7), der in der Doppelschicht (3) enthalten ist, zwischen 3,8 % und 8,5 % liegt.

26. Vesikel (1) gemäß Anspruch 25,
**dadurch gekennzeichnet,**
**dass** als Massenprozentanteil, bezogen auf die Gesamtmasse des Vesikels (1), der hydrophobe innere Hohlraum (2) aus
- mindestens einer hydrophoben neutralen Verbindung und/oder mindestens einer hydrophoben Verbindung von Interesse, deren Massenprozentanteil zwischen 70 % und 89 % liegt, besteht, und außerdem:
- mindestens ein Antioxidans, dessen Massenprozentanteil zwischen 0 % und 4 % liegt, umfasst.

27. Vesikel (1) gemäß Anspruch 25,
**dadurch gekennzeichnet,**
**dass** als Massenprozentanteil, bezogen auf die Gesamtmasse des Vesikels (1), die Doppelschicht (3) aus amphiphilen Lipiden (6) aus:
- amphiphilen Lipiden (6), deren Massenprozentanteil zwischen 10 % und 22,5 % liegt, besteht,
und außerdem:
- mindestens einen Emulgator, dessen Massenprozentanteil zwischen 0 %und 4,5 % liegt, umfasst,
und außerdem:
- mindestens einen Stabilisator, dessen Massenprozentanteil zwischen 0 % und 1 % liegt, umfasst.

28. Vesikel (1) gemäß Anspruch 25,
**dadurch gekennzeichnet,**
**dass** als Massenprozentanteil, bezogen auf die Gesamtmasse des Vesikels (1), der hydrophile interstitielle Raum (7) aus:
- Wasser, dessen Massenprozentanteil zwischen 3,8 % und 8,5 % liegt, besteht
und außerdem
- mindestens ein Mittel, das eine Barriere gegen Sauerstoff bildet, dessen Massenprozentanteil zwischen 0 % und 0,2 % liegt, und/oder
- mindestens ein Mittel, das fähig ist, die Steifigkeit der Lipid-Doppelschicht zu erhöhen und/oder die Verfügbarkeit der hydrophilen Phase zu verringern, dessen Massenprozentanteil zwischen 0 % und 2,135 % liegt, umfasst.

29. Vesikel (1) gemäß Anspruch 28,
**dadurch gekennzeichnet,**
**dass** als Massenprozentanteil, bezogen auf die Gesamtmasse des Vesikels (1), der hydrophile interstitielle Raum (7) umfasst:
- native oder vernetzte Stärke, deren Massenprozentanteil zwischen 0 % und 1,5 % liegt,
- und/oder Maltodextrine, deren Massenprozentanteil zwischen 0 % und 0,3 % liegt,
- und/oder Akazien-Gummi, dessen Massenprozentanteil zwischen 0 %und 0,3 liegt,
- und/oder Guar-Gummi, dessen Massenprozentanteil zwischen 0 % und 0,02 % liegt
- und/oder Xanthan-Gummi, dessen Massenprozentanteil zwischen 0 % und 0,015 % liegt.

30. Vesikel (1) gemäß einem der Ansprüche 25 bis 29,
**dadurch gekennzeichnet,**
**dass** als Massenprozentanteil bezogen auf die Gesamtmasse des Vesikels (1):
- der hydrophobe innere Hohlraum (2) aus mindestens einer hydrophoben neutralen Verbindung und/oder mindestens einer hydrophoben Verbindung von Interesse, deren Massenprozentanteil 75 % beträgt, und mindestens einem Antioxidans, dessen Massenprozentanteil 3,5 % beträgt, besteht,
- die Doppelschicht (3) aus amphiphilen Lipiden (6) aus amphiphilen Lipiden, deren Massenprozentanteil zwischen 13 % und 16 % liegt, besteht und mindestens einen Emulgator, dessen Massenprozentanteil 3,5 % beträgt, und mindestens einen Stabilisator, dessen Massenprozentanteil 0,8 % beträgt, umfasst,
- der hydrophile interstitielle Raum (7) aus Wasser besteht, dessen Massenprozentanteil 4 % beträgt, er mindestens ein Mittel umfasst, das eine Barriere gegen Sauerstoff bildet, dessen Massenprozentanteil 0,11 % beträgt, und er mindestens ein Mittel umfasst, das geeignet ist, die Steifigkeit der Lipid-Doppelschicht zu erhöhen und/oder die Verfügbarkeit der hydrophilen Phase zu verringern, dessen Massenprozentanteil 0,705 % beträgt.

31. Vesikel (1) gemäß Anspruch 30,
**dadurch gekennzeichnet,**
**dass** als Massenprozentanteil, bezogen auf die Gesamtmasse des Vesikels (1), der hydrophile interstitielle Raum (7) umfasst:
- native oder vernetzte Stärke, deren Massenprozentanteil 0,08 % beträgt,
- Maltodextrine, deren Massenprozentanteil 0,3 % beträgt,
- Akazien-Gummi, dessen Massenprozentanteil 0,3 % beträgt,
- Guar-Gummi, dessen Massenprozentanteil 0,015 % beträgt,
- Xanthan-Gummi, dessen Massenprozentanteil 0,01 % beträgt.

32. Fettphase (4) eines Produktes,
**dadurch gekennzeichnet,**
**dass** sie mindestens ein Vesikel (1), wie es in einem der Ansprüche 1 bis 31 definiert ist, umfasst.

33. Kosmetische oder pharmazeutische Produkte,
**dadurch gekennzeichnet,**
**dass** sie mindestens ein Vesikel (1), wie es in einem der Ansprüche 1 bis 31 definiert ist, umfassen.

34. Alimentäre Produkte,
**dadurch gekennzeichnet,**
**dass** sie mindestens ein Vesikel (1), wie es in den Ansprüchen 1 bis 31 definiert ist, umfassen.

35. Kosmetische oder pharmazeutische Produkte gemäß Anspruch 33,
**dadurch gekennzeichnet,**
**dass** sie in ihrer Fettphase (4) mindestens ein Vesikel (1), wie es in den Ansprüchen 1 bis 31 definiert ist, umfassen.

36. Alimentäre Produkte gemäß Anspruch 34,
**dadurch gekennzeichnet,**
**dass** sie in ihrer Fettphase (4) mindestens ein Vesikel (1), wie es in den Ansprüchen 1 bis 31 definiert ist, umfassen.

37. Verfahren zur Herstellung von Vesikeln (1), wie sie in einem der Ansprüche 1 bis 31 definiert sind, umfassend die folgenden Stufen:
- Stufe A: Ausbreiten (Spreitung) der amphiphilen Lipide (6),
- Stufe B: Zusatz einer hydrophilen Phase, aus der der hydrophile interstitielle Raum (7) gebildet werden wird,
- Stufe C: Anordnen, um Lipid-Doppelschichten (3) zu erhalten,
- Stufe D: Entfernung des Überschusses der hydrophilen Phase (7), um hydratisierte Phospholipidfilme zu erhalten,
- Stufe E: Zusatz einer hydrophoben Phase, die den hydrophoben inneren Hohlraum (2) bilden wird,
- Stufe F: starkes Rühren.

38. Verfahren gemäß Anspruch 37,
**dadurch gekennzeichnet, dass**:
- in der Stufe A die Ausbreitung der amphiphilen Lipide (6) an der Wand eines Reaktors in Rotation oder an einer ebenen Oberfläche durchgeführt wird,
- in der Stufe B die hydrophile Phase in den Reaktor gegeben wird oder sie auf der ebenen Oberfläche versprüht wird oder die ebene Oberfläche in ein Bad der hydrophilen Phase eingetaucht wird,
- in der Stufe E die hydrophobe Phase in den Reaktor gegeben wird oder in ein Aufnahmegefäß gegeben wird, dann auf die hydratisierten Phospholipidfilme (5) gegeben wird.

39. Verfahren gemäß dem einem oder dem anderen der Ansprüche 37 und 38,
**dadurch gekennzeichnet,**
**dass** in der Stufe E die hydrophobe Phase außerdem aus mindestens einer hydrophoben Verbindung von Interesse gebildet wird.

40. Verfahren gemäß Anspruch 39,
**dadurch gekennzeichnet,**
**dass** die hydrophobe Verbindung von Interesse ein Fischöl ist.

41. Verfahren gemäß einem der Ansprüche 37 bis 40,
**dadurch gekennzeichnet,**
**dass** es eine zusätzliche Stufe:
- Stufe G: Dekantieren und Gewinnen der Vesikel, umfasst.

42. Verfahren gemäß Anspruch 41,
**dadurch gekennzeichnet,**
**dass** es eine zusätzliche Stufe:
- Stufe H: Einführung der Vesikel in die Fettphase (4) eines Produkts, umfasst.

43. Verwendung von Vesikeln (1), wie sie in einem der Ansprüche 1 bis 31 definiert sind,
als Transport-Abgabe-System für mindestens eine hydrophobic Verbindung von Interesse und gegebenenfalls für mindestens eine hydrophile Verbindung von Interesse.

44. Verwendung gemäß Anspruch 43,
**dadurch gekennzeichnet,**
**dass** die hydrophile Verbindung von Interesse ausgewählt ist aus hydrophilen pharmazeutischen und/oder kosmetischen Wirkstoffen oder Ingredienzien oder hydrophilen alimentären Wirkstoffen oder Ingredienzien und sie sich in dem hydrophilen interstitiellen Raum (7), der in der Doppelschicht (3) aus amphiphilen Lipiden (6) enthalten ist, befindet.

45. Verwendung gemäß Anspruch 43,
**dadurch gekennzeichnet,**
**dass** die hydrophobe Verbindung von Interesse ausgewählt ist aus hydrophoben pharmazeutischen und/oder kosmetischen Wirkstoffen oder Ingredienzien oder hydrophoben alimentären Wirkstoffen oder Ingredienzien und sie sich in dem hydrophoben inneren Hohlraum (2) befindet.

46. Verwendung gemäß Anspruch 45,
**dadurch gekennzeichnet,**
**dass** die hydrophobe Verbindung von Interesse ein Öl ist, das aus ätherischen Ölen, marinen mehrfach ungesättigten Ölen oder pflanzlichen mehrfach ungesättigten Ölen ausgewählt ist.

47. Verwendung gemäß Anspruch 46,
**dadurch gekennzeichnet,**
**dass** die hydrophobe Verbindung von Interesse ein Fischöl ist.

48. Verwendung von Vesikeln (1), wie sie gemäß einem der Ansprüche 1 bis 31 definiert sind,
für den Schutz mindestens einer Verbindung von Interesse besonders hydrophobe gegen Hydrolyse und/oder Oxidation.

49. Verwendung gemäß Anspruch 48,
**dadurch gekennzeichnet,**
**dass** die Verbindung von Interesse ein Öl, ausgewählt aus ätherischen Ölen, marinen mehrfach ungesättigten Ölen oder pflanzlichen mehrfach ungesättigten Ölen, ist.

50. Verwendung gemäß Anspruch 49,
**dadurch gekennzeichnet,**
**dass** die Verbindung von Interesse ein Fischöl ist.

51. Verwendung von Vesikeln (1), wie sie gemäß einem der Ansprüche 1 bis 31 definiert sind,
um Produkte anzureichern, die aus einer Fettphase (4) bestehen, an mindestens einer hydrophoben Verbindung von Interesse, die eine hohe Konzentration an ungesättigten und/oder Omega-3/6-Fettsäuren aufweist, anzureichern.

52. Verwendung gemäß Anspruch 51,
**dadurch gekennzeichnet,**
**dass** die hydrophobe Verbindung von Interesse ein Fischöl ist.

53. Verwendung von Vesikeln (1), wie sie in einem der Ansprüche 1 bis 31 definiert sind, umfassend im hydrophoben inneren Hohlraum (2) eine hydrophobe Verbindung von Interesse, die ein Öl ist, um die Entwicklung von unangenehmen Gerüchen und/oder Geschmacksnoten, die durch das Öl freigesetzt werden, zu verhindern, in alimentären, kosmetischen oder pharmazeutischen Produkten.

54. Verwendung gemäß Anspruch 53,
**dadurch gekennzeichnet,**
**dass** das Öl ein Öl, ausgewählt aus ätherischen Ölen, marinen mehrfach ungesättigten Ölen oder pflanzlichen mehrfach ungesättigten Ölen, ist.

55. Verwendung gemäß Anspruch 54,
**dadurch gekennzeichnet,**
**dass** das Öl ein Fischöl ist.

## Claims

1. Vesicle (1) which is hydrophobic in nature and can be incorporated into a fatty phase, **characterised in that** it comprises:
- an oily hydrophobic inner cavity (2) composed of at least one hydrophobic neutral compound or of at least one hydrophobic compound of interest or of both at least one hydrophobic neutral compound and at least one hydrophobic compound of interest, and
- at least one bilayer (3) composed of two layers of amphiphilic lipids (6) that define between them a hydrophilic interstitial space (7), and
- the hydrophobic neutral compound is a neutral oil chosen from groundnut oil *(Arachis hypogea),* adjouaba oil (*Haematostaphis barteri*), avocado oil *(Persea americana),* babassu oil *(Orbignya oleifera),* shea butter oil (*Vitallaria paradoxa*), cocoa butter oil *(Theobroma cacao),* chufa oil (*Cyperus esculentus*), crambe oil *(Crambe abyssinica),* cuphea oil *(Cuphea tolucana, Cuphea wrightii, Cuphea laminuligena, Cuphea ilavea* or *Cuphea leptopoda),* maple oil *(Acer saccharum* or *Acer saccharinum*), gokhru oil *(Tribulus terrestris),* hazelnut oil (*Corylus avellana),* coconut oil *(Cocus nocifera),* Macadamia nut oil *(Macadamia tetraphylla),* Cambodia nut oil *(Irvingia malayana),* pili nut oil (*Canarium ovatum*), apricot kernel oil (*Prunus armeniaca*), olive oil *(Olea europaea),* palm oil *(Elaeis guineensis),* palm kernel oil (*Elaeis guineensis*), mango seed oil *(Mangifer indica),* pistachio oil *(Pistacia atlantica),* or fractions of those oils (such as, for example, palm or palm kernel oleins and stearins), said oil being on its own or in a mixture,
- the hydrophobic compound of interest is chosen from hydrophobic pharmaceutical and/or cosmetic active principles or ingredients or hydrophobic food active principles or ingredients or
- is an oil chosen from essential oils, polyunsaturated marine oils or polyunsaturated plant oils, on their own or in a mixture.

2. Vesicle (1) according to claim 1, **characterised in that** the polyunsaturated marine oil is chosen from fish oils.

3. Vesicle (1) according to claim 1 or 2, **characterised in that** the essential oil is chosen from lemon essential oil, lavender essential oil, eucalyptus essential oil, camphor essential oil, sage essential oil, thyme essential oil, clove essential oil.

4. Vesicle (1) according to any one of claims 1 to 3, **characterised in that** the polyunsaturated plant oil is an oil rich in omega-3 fatty acids or in omega-6 fatty acids.

5. Vesicle (1) according to any one of claims 1 to 4, **characterised in that** when the number of bilayers (3) of amphiphilic lipids (6) is greater than or equal to 2, said bilayers (3) are concentric.

6. Vesicle (1) according to any one of claims 1 to 4, **characterised in that** when the number of bilayers (3) of amphiphilic lipids (6) is greater than or equal to 3, said bilayers (3) are juxtaposed.

7. Vesicle (1) according to any one of claims 1 to 4, **characterised in that** when the number of bilayers (3) of amphiphilic lipids (6) is greater than or equal to 4, said bilayers (3) are concentric and juxtaposed.

8. Vesicle (1) according to any one of claims 1 to 7, **characterised in that** the amphiphilic lipids (6) have their hydrophilic part oriented head-to-head towards the inside of the bilayer (3), thus delimiting the hydrophilic interstitial space (7).

9. Vesicle (1) according to any one of claims 1 to 8, **characterised in that** the amphiphilic lipids (6) of the bilayer (3) are phospholipids.

10. Vesicle (1) according to claim 9, **characterised in that** the amphiphilic lipids (6) of the bilayer (3) are soy lecithins.

11. Vesicle (1) according to any one of claims 1 to 10, **characterised in that** the bilayer (3) further comprises at least one emulsifier, such as those chosen from the group of the monoglycerides or diglycerides of fatty acids.

12. Vesicle (1) according to any one of claims 1 to 11, **characterised in that** the bilayer (3) further comprises at least one stabilising compound, such as those chosen from the compounds of the tocopherol type, such as alpha-tocopherol, or of the sterol type, such as cholesterol.

13. Vesicle (1) according to any one of claims 1 to 12, **characterised in that** the hydrophilic interstitial space (7) contained in the bilayer (3) is composed of water.

14. Vesicle (1) according to any one of claims 1 to 13, **characterised in that** the hydrophilic interstitial space (7) defined by the bilayer (3) further comprises at least one oxygen barrier agent.

15. Vesicle (1) according to claim 14, **characterised in that** the at least one oxygen barrier agent is chosen from hydrocolloid gels of low hydration, beeswax, lactoserum protein isolate, soy protein isolate, β-lactoglobulin, casein, casein salts such as sodium caseinate, sorbitol, glycerol, taken on their own or in a mixture.

16. Vesicle (1) according to any one of claims 1 to 15, **characterised in that** the hydrophilic interstitial space (7) defined by the bilayer (3) further comprises at least one agent capable of increasing the rigidity of said bilayer (3) and/or of reducing the availability of the hydrophilic phase.

17. Vesicle (1) according to claim 16, **characterised in that** the at least one agent capable of increasing the rigidity of said bilayer (3) and/or of reducing the availability of the hydrophilic phase is chosen from hydrocolloids such as natural starch (wheat starch, rice starch, corn starch, paprika starch, potato starch), crosslinked starch, pregelatinised starch, amylopectin, or starch degradation products such as maltodextrins of different degrees of hydrolysis, gums such as acacia gums, guar gums, xanthan gums, tragacanth gums, karaya gums, tara gums, gellan gums, sorbitol, sorbitol syrup, mannitol, glycerol, glucomannan, polyoxyethylene (8) stearate, polyoxyethylene (40) stearate, polyoxyethylene sorbitan monolaurate (polysorbate 20), polyoxyethylene sorbitan monooleate (polysorbate 80), polyoxyethylene sorbitan monopalmitate (polysorbate 40), polyoxyethylene sorbitan monostearate (polysorbate 60), poloxyethylene sorbitan tristearate (polysorbate 65), pectin, amidated pectin, ammonium phosphatides, sucrose acetate isobutyrate, glycerol esters of wood resin, alginic acid, sodium alginate, potassium alginate, ammonium alginate, calcium alginate or 1,2-propanediol alginate, agar-agar, carrageenans, processed euchema seaweed or locust bean gum, taken on their own or in a mixture.

18. Vesicle (1) according to any one of claims 1 to 17, **characterised in that** the hydrophilic interstitial space (7) defined by the bilayer (3) further comprises at least one hydrophilic compound of interest.

19. Vesicle (1) according to claim 18, **characterised in that** the hydrophilic compound of interest is chosen from hydrophilic pharmaceutical and/or cosmetic active principles or ingredients or hydrophilic food active principles or ingredients.

20. Vesicle (1) according to claim 19, **characterised in that** the hydrophilic compound of interest is chosen from anti-tumour agents (adriamycin, actimycin, mitomycin, 1-β-arabinofuranosylcytosine, cisplatin), antivirals (interferon), aminoglucosides (gentamicin), antibiotics (β-lactam, sulbenicillin, cefotiam, cefmenoxime), peptide hormones (TRH, leuprolide, insulin), immunopotentiating agents (muramyl dipeptide, muramyl tripeptide), proteins (immunoglobulins, toxins), anti-allergic agents, antimycotic agents, cytostatic agents or diagnostic agents, anxiolytics, contraceptives, sedatives, mineral salts (calcium, chlorine, magnesium, phosphorus, potassium, sodium, sulfur), oligo-elements (aluminium, bromine, copper, cobalt, iron, fluorine, manganese, molybdenum, iodine, selenium, silicon, vanadium, zinc), glucides (such as glucose, galactose, mannose, maltose, maltotriose), amino acids (alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine), peptides, proteins, hydrosoluble vitamins, flavourings (lemon, vanilla, strawberry, coffee, hazelnut, raspberry, etc.), polyols.

21. Vesicle (1) according to any one of claims 1 to 20, **characterised in that** the hydrophobic compound of interest is chosen from paracetamol, pindolol, probucol.

22. Vesicle (1) according to any one of claims 1 to 20, **characterised in that** the hydrophobic compound of interest is chosen from antioxidants such as rosemary extract, green tea extract, a mixture of tocopherols, free fatty acids such as EPA, DHA, arachidonic acid, essential fatty acids such as linoleic acid, alpha-linolenic acid, plant sterols, plant stanols, policosanols, liposoluble vitamins such as vitamin A, D, E, K, colourings such as carotenoids, flavonoids, aromatic compounds such as diacetyl, aromatic edible oils (virgin walnut oil, virgin olive oil, virgin hazelnut oil).

23. Vesicle (1) according to any one of claims 1 to 22, **characterised in that** the hydrophobic inner cavity further comprises at least one antioxidant.

24. Vesicle (1) according to claim 23, **characterised in that** the at least one antioxidant is chosen from tocopherols in the form of a natural mixture, or synthetic alpha-tocopherol, or synthetic gamma-tocopherol, or synthetic delta-tocopherol, ascorbyl palmitate, ascorbyl stearate, propyl gallate, octyl gallate, dodecyl gallate, BHA (butylhydroxyanisole), BHT (butylhydroxytoluene), EDTA (ethylenediaminetetraacetate), natural rosemary extract, green tea extract, polyphenols, taken on their own or in a mixture.

25. Vesicle (1) according to any one of claims 1 to 24, **characterised in that**, in percent by mass, based on the total mass of the vesicle (1):
- the percentage by mass of the hydrophobic inner cavity (2) is from 70 to 89%,
- the percentage by mass of the bilayer (3) of amphiphilic lipids (6) is from 10 to 22.5%,
- the percentage by mass of the hydrophilic interstitial space (7) contained in the bilayer (3) is from 3.8 to 8.5%.

26. Vesicle (1) according to claim 25, **characterised in that**, in percent by mass, based on the total mass of the vesicle (1), the hydrophobic inner cavity (2) is composed of:
- at least one hydrophobic neutral compound and/or at least one hydrophobic compound of interest, the percentage by mass of which is from 70 to 89%,
and further comprises:
- at least one antioxidant, the percentage by mass of which is from 0 to 4%.

27. Vesicle (1) according to claim 25, **characterised in that**, in percent by mass, based on the total mass of the vesicle (1), the bilayer (3) of amphiphilic lipids (6) is composed of:
- amphiphilic lipids (6), the percentage of which is from 10 to 22.5%,
and further comprises:
- at least one emulsifier, the percentage by mass of which is from 0 to 4.5%,
and further comprises:
- at least one stabiliser, the percentage by mass of which is from 0 to 1 %.

28. Vesicle (1) according to claim 25, **characterised in that**, in percent by mass, based on the total mass of the vesicle (1), the hydrophilic interstitial space (7) is composed of:
- water, the percentage by mass of which is from 3.8 to 8.5%,
and further comprises
- at least one oxygen barrier agent, the percentage by mass of which is from 0 to 0.2%, and/or
- at least one agent capable of increasing the rigidity of the lipid bilayer and/or of reducing the availability of the hydrophilic phase, the percentage by mass of which is from 0 to 2.135%.

29. Vesicle (1) according to claim 28, **characterised in that**, in percent by mass, based on the total mass of the vesicle (1), the hydrophilic interstitial space (7) comprises
- natural or crosslinked starch, the percentage by mass of which is from 0 to 1.5%,
- and/or maltodextrins, the percentage by mass of which is from 0 to 0.3%,
- and/or acacia gum, the percentage by mass of which is from 0 to 0.3%,
- and/or guar gum, the percentage by mass of which is from 0 to 0.02%,
- and/or xanthan gum, the percentage by mass of which is from 0 to 0.015%.

30. Vesicle (1) according to any one of claim 25 to 29, **characterised in that**, in percent by mass, based on the total mass of the vesicle (1):
- the hydrophobic inner cavity (2) is composed of at least one hydrophobic neutral compound and/or at least one hydrophobic compound of interest, the percentage by mass of which is 75%, and at least one antioxidant, the percentage by mass of which is 3.5%,
- the bilayer (3) of amphiphilic lipids (6) is composed of amphiphilic lipids, the percentage by mass of which is from 13 to 16%, and comprises at least one emulsifier, the percentage by mass of which is 3.5%, and at least one stabiliser, the percentage by mass of which is 0.8%,
- the hydrophilic interstitial space (7) is composed of water, the percentage by mass of which is 4%, it comprises at least one oxygen barrier agent, the percentage by mass of which is 0.11%, and it comprises at least one agent capable of increasing the rigidity of the lipid bilayer and/or of reducing the availability of the hydrophilic phase, the percentage by mass of which agent is 0.705%.

31. Vesicle (1) according to claim 30, **characterised in that**, in percent by mass, based on the total mass of the vesicle (1), the hydrophilic interstitial space (7) comprises:
- natural or crosslinked starch, the percentage by mass of which is 0.08%,
- maltodextrins, the percentage by mass of which is 0.3%,
- acacia gum, the percentage by mass of which is 0.3%,
- guar gum, the percentage by mass of which is 0.015%,
- xanthan gum, the percentage by mass of which is 0.01%.

32. Fatty phase (4) of a product, **characterised in that** it comprises at least one vesicle (1) as defined in any one of claims 1 to 31.

33. Cosmetic or pharmaceutical products, **characterised in that** they comprise at least one vesicle (1) as defined in claims 1 to 31.

34. Food products, **characterised in that** they comprise at least one vesicle (1) as defined in claims 1 to 31.

35. Cosmetic or pharmaceutical products according to claim 33, **characterised in that** they comprise in their fatty phase (4) at least one vesicle (1) as defined in claims 1 to 31.

36. Food products according to claim 34, **characterised in that** they comprise in their fatty phase (4) at least one vesicle (1) as defined in claims 1 to 31.

37. Process for the preparation of vesicles (1) as defined according to any one of claims 1 to 31, comprising the following steps:
- step A: spreading of the amphiphilic lipids (6),
- step B: addition of a hydrophilic phase which will constitute the hydrophilic interstitial space (7),
- step C: resting in order to obtain lipid bilayers (3),
- step D: removal of excess hydrophilic phase (7) to obtain hydrated phospholipid films (5),
- step E: addition of a hydrophobic phase which will constitute the hydrophobic inner cavity (2),
- step F: vigorous stirring.

38. Process according to claim 37, **characterised in that**:
- in step A, the spreading of the amphiphilic lipids (6) is carried out on the wall of a rotating reactor or on a flat surface,
- in step B, the hydrophilic phase is added to the reactor or is sprayed onto the flat surface, or the flat surface is immersed in a bath of hydrophilic phase,
- in step E, the hydrophobic phase is added to the reactor or placed in a container and then added over the hydrated phospholipid films (5).

39. Process according to either claim 37 or claim 38, **characterised in that** in step E the hydrophobic phase is further composed of at least one hydrophobic compound of interest.

40. Process according to claim 39, **characterised in that** the hydrophobic compound of interest is a fish oil.

41. Process according to any one of claims 37 to 40, **characterised in that** it comprises an additional step:
- step G: separation and recovery of the vesicles.

42. Process according to claim 41, **characterised in that** it comprises an additional step:
- step H: introduction of the vesicles into the fatty phase (4) of a product.

43. Use of vesicles (1) as defined according to any one of claims 1 to 31 as a transport-delivery system for at least one hydrophobic compound of interest, and eventually for at least one hydrophilic compound of interest.

44. Use according to claim 43, **characterised in that** the hydrophilic compound of interest is chosen from hydrophilic pharmaceutical and/or cosmetic active principles or ingredients or hydrophilic food active principles or ingredients, and it is located in the hydrophilic interstitial space (7) contained in the bilayer (3) of amphiphilic lipids (6).

45. Use according to claim 43, **characterised in that** the hydrophobic compound of interest is chosen from hydrophobic pharmaceutical and/or cosmetic active principles or ingredients or hydrophobic food active principles or ingredients, and it is located in the hydrophobic inner cavity (2).

46. Use according to claim 45, **characterised in that** the hydrophobic compound of interest is an oil chosen from essential oils, polyunsaturated marine oils or polyunsaturated plant oils.

47. Use according to claim 46, **characterised in that** the hydrophobic compound of interest is a fish oil.

48. Use of vesicles (1) as defined according to any one of claims 1 to 31 for protecting at least one compound of interest, especially one hydrophobic compound of interest, against hydrolysis and/or oxidation.

49. Use according to claim 48, **characterised in that** the compound of interest is an oil chosen from essential oils, polyunsaturated marine oils or polyunsaturated plant oils.

50. Use according to claim 49, **characterised in that** the compound of interest is a fish oil.

51. Use of vesicles (1) as defined according to any one of claims 1 to 31 for enriching products composed of a fatty phase (4) with at least one hydrophobic compound of interest having a high content of unsaturated fatty acids and/or omega-3/6 fatty acids.

52. Use according to claim 51, **characterised in that** the hydrophobic compound of interest is a fish oil.

53. Use in food products, cosmetic products or pharmaceutical products of vesicles (1) as defined according to any one of claims 1 to 31 that comprise in the hydrophobic inner cavity (2) a hydrophobic compound of interest which is an oil, for preventing the development of unpleasant odours and/or tastes released by said oil.

54. Use according to claim 53, **characterised in that** the oil is an oil chosen from essential oils, polyunsaturated marine oils or polyunsaturated plant oils.

55. Use according to claim 54, **characterised in that** the oil is a fish oil.
